# EUROPEAN PATENT APPLICATION

(11) **EP 4 273 171 A1**
(43) Date of publication of application: **08.11.2023**
(21) Application number: 21914404.5
(22) Date of filing: 28.12.2021
(51) Int. Cl.: C07K 16/30, C07K 16/00, A61K 39/395, A61K 39/00, C12P 21/08

(54) **ANTIBODY CAPABLE OF BINDING TO TROP2, AND USE THEREOF**

(30) Priority: 30.12.2020 CN 202011611968
(71) Applicant: Nona Biosciences (Suzhou) Co., Ltd., Suzhou, Jiangsu 215123 (CN)
(72) Inventor: LV, Qiang, Suzhou, Jiangsu 215123 (CN); HUANG, Bing, Suzhou, Jiangsu 215123 (CN); RONG, Yiping, Suzhou, Jiangsu 215123 (CN)
(74) Representative: Mewburn Ellis LLP
(86) International application number: PCT/CN2021/142095
(87) International publication number: WO 2022/143670

(57) **Abstract**

Provided is an isolated monoclonal antibody capable of specifically binding to Trop2. Also provided are a nucleic acid molecule encoding the antibody, as well as an expression vector, a host cell, and a method for expressing the antibody. Further provided are an immunoconjugate, a bispecific molecule, a chimeric antigen receptor, an oncolytic virus, and a pharmaceutical composition containing the antibody, and a diagnostic or therapeutic method using the antibody.

## Description

### TECHNICAL FIELD

The present invention relates to an antibody specifically binding to Trop2, and a preparation method therefor and use thereof, in particular to use thereof in the diagnosis, prevention, and treatment of a Trop2-related disease, wherein the disease includes tumors, e.g., breast cancer, gastric cancer, pancreatic cancer, ovarian cancer, intestinal cancer, and the like.

### BACKGROUND

Trophoblast cell-surface antigen 2 (Trop2), also known as tumor-associated calcium signal transducer 2 (TACSTD2), membrane component, chromosome 1, surface marker 1 (M1S1), epithelial glycoprotein 1(EGP1), or gastrointestinal antigen 733-1 (GA733-1), is a type I transmembrane cell surface glycoprotein.

Encoded by intronless genes, Trop2 has 323 amino acid residues in total. The protein can transduce intracellular calcium signals and contains a conserved phosphatidylinositol 4,5-bisphosphate (PIP(2)) binding motif and a serine phosphorylation site that interacts with protein kinase C. Trop2 also interacts with IGF-1, Claudin-1, Claudin-7, and cyclin D1. For example, Trop2 activates CREB1, NF-κB, STAT1, and STAT3 through the cyclin D1 and the ERK-MAPK pathway, which contributes to the progression of tumor cells. Trop2 is expressed in many normal tissues and plays an important role in organogenesis and stem cell maintenance. Meanwhile, Trop2 is also highly expressed in cancer cells of many people, such as breast, gastric, pancreatic, and ovarian cancer cells, and overexpression of Trop2 is related to increased tumor growth, tumor invasiveness, tumor metastasis, and poor prognosis. For example, low expression of Trop2 in intestinal and ovarian tumor cells inhibits cell proliferation. The overexpression of Trop2 increases migration of pancreatic cancer cells. Thus, Trop2 is considered a potential therapeutic target.

Studies have shown that the Trop2 antibody inhibited the tumor growth *in vivo* and reduced the migration of intestinal cancer cells and breast cancer cells *in vivo.* Sacituzumab govitecan (IMMU-132), a Trop2 antibody-SN-38 conjugate, has been demonstrated to be effective in several tumor cell line transplantation models, and is undergoing clinical trials in patients with refractory triple-negative breast cancer, metastatic urothelial cancer, and metastatic castration-resistant prostate cancer, with neutropenia and diarrhea being the most common side effects.

Therefore, there is an urgent need in the art to develop a Trop2 monoclonal antibody with a better anti-tumor effect and low toxicity.

### SUMMARY

The present invention aims to provide a Trop2 monoclonal antibody with a better anti-tumor effect and low toxicity.

In a first aspect of the present invention, provided is an antibody or an antigen-binding fragment against Trop-2 comprising complementarity determining regions (CDRs) as follows:
(a) an HCDR1 or a variant of a sequence thereof, an HCDR2 or a variant of a sequence thereof, and an HCDR3 or a variant of a sequence thereof comprised in a heavy chain variable region (VH) set forth in any one of SEQ ID NOs: 192-220; and/or
(b) an LCDR1 or a variant of a sequence thereof, an LCDR2 or a variant of a sequence thereof, and an LCDR3 or a variant of a sequence thereof comprised in a light chain variable region (VL) set forth in any one of SEQ ID NOs: 221-240;
   preferably, the variant of the sequence is a CDR having one or several amino acid substitutions, deletions, or additions (e.g., 1, 2 or 3 amino acid substitutions, deletions, or additions) compared to a CDR from which the variant is derived; preferably, the substitutions are conservative substitutions.

In another preferred embodiment, the antibody or the antigen-binding protein comprises a heavy chain variable region (VH) having combination of complementarity determining regions (CDRs) selected from the group consisting of:
(1) a CDR1 set forth in SEQ ID NO: 19, a CDR2 set forth in SEQ ID NO: 47, and a CDR3 set forth in SEQ ID NO: 86;
(2) a CDR1 set forth in SEQ ID NO: 20, a CDR2 set forth in SEQ ID NO: 48, and a CDR3 set forth in SEQ ID NO: 87;
(3) a CDR1 set forth in SEQ ID NO: 20, a CDR2 set forth in SEQ ID NO: 48, and a CDR3 set forth in SEQ ID NO: 87;
(4) a CDR1 set forth in SEQ ID NO: 21, a CDR2 set forth in SEQ ID NO: 49, and a CDR3 set forth in SEQ ID NO: 88;
(5) a CDR1 set forth in SEQ ID NO: 21, a CDR2 set forth in SEQ ID NO: 50, and a CDR3 set forth in SEQ ID NO: 89;
(6) a CDR1 set forth in SEQ ID NO: 22, a CDR2 set forth in SEQ ID NO: 51, and a CDR3 set forth in SEQ ID NO: 90;
(7) a CDR1 set forth in SEQ ID NO: 23, a CDR2 set forth in SEQ ID NO: 52, and a CDR3 set forth in SEQ ID NO: 91;
(8) a CDR1 set forth in SEQ ID NO: 24, a CDR2 set forth in SEQ ID NO: 53, and a CDR3 set forth in SEQ ID NO: 92;
(9) a CDR1 set forth in SEQ ID NO: 25, a CDR2 set forth in SEQ ID NO: 54, and a CDR3 set forth in SEQ ID NO: 87;
(10) a CDR1 set forth in SEQ ID NO: 26, a CDR2 set forth in SEQ ID NO: 51, and a CDR3 set forth in SEQ ID NO: 93;
(11) a CDR1 set forth in SEQ ID NO: 24, a CDR2 set forth in SEQ ID NO: 53, and a CDR3 set forth in SEQ ID NO: 92;
(12) a CDR1 set forth in SEQ ID NO: 21, a CDR2 set forth in SEQ ID NO: 50, and a CDR3 set forth in SEQ ID NO: 89;
(13) a CDR1 set forth in SEQ ID NO: 27, a CDR2 set forth in SEQ ID NO: 55, and a CDR3 set forth in SEQ ID NO: 94;
(14) a CDR1 set forth in SEQ ID NO: 27, a CDR2 set forth in SEQ ID NO: 55, and a CDR3 set forth in SEQ ID NO: 95;
(15) a CDR1 set forth in SEQ ID NO: 27, a CDR2 set forth in SEQ ID NO: 55, and a CDR3 set forth in SEQ ID NO: 94;
(16) a CDR1 set forth in SEQ ID NO: 19, a CDR2 set forth in SEQ ID NO: 52, and a CDR3 set forth in SEQ ID NO: 96;
(17) a CDR1 set forth in SEQ ID NO: 27, a CDR2 set forth in SEQ ID NO: 55, and a CDR3 set forth in SEQ ID NO: 95;
(18) a CDR1 set forth in SEQ ID NO: 27, a CDR2 set forth in SEQ ID NO: 55, and a CDR3 set forth in SEQ ID NO: 95;
(19) a CDR1 set forth in SEQ ID NO: 27, a CDR2 set forth in SEQ ID NO: 55, and a CDR3 set forth in SEQ ID NO: 97;
(20) a CDR1 set forth in SEQ ID NO: 27, a CDR2 set forth in SEQ ID NO: 55, and a CDR3 set forth in SEQ ID NO: 94;
(21) a CDR1 set forth in SEQ ID NO: 27, a CDR2 set forth in SEQ ID NO: 55, and a CDR3 set forth in SEQ ID NO: 94;
(22) a CDR1 set forth in SEQ ID NO: 27, a CDR2 set forth in SEQ ID NO: 56, and a CDR3 set forth in SEQ ID NO: 95;
(23) a CDR1 set forth in SEQ ID NO: 28, a CDR2 set forth in SEQ ID NO: 57, and a CDR3 set forth in SEQ ID NO: 98;
(24) a CDR1 set forth in SEQ ID NO: 27, a CDR2 set forth in SEQ ID NO: 55, and a CDR3 set forth in SEQ ID NO: 95;
(25) a CDR1 set forth in SEQ ID NO: 29, a CDR2 set forth in SEQ ID NO: 58, and a CDR3 set forth in SEQ ID NO: 99;
(26) a CDR1 set forth in SEQ ID NO: 27, a CDR2 set forth in SEQ ID NO: 55, and a CDR3 set forth in SEQ ID NO: 94;
(27) a CDR1 set forth in SEQ ID NO: 23, a CDR2 set forth in SEQ ID NO: 59, and a CDR3 set forth in SEQ ID NO: 100;
(28) a CDR1 set forth in SEQ ID NO: 30, a CDR2 set forth in SEQ ID NO: 51, and a CDR3 set forth in SEQ ID NO: 101;
(29) a CDR1 set forth in SEQ ID NO: 31, a CDR2 set forth in SEQ ID NO: 59, and a CDR3 set forth in SEQ ID NO: 100; and
(30) a CDR1 set forth in SEQ ID NO: 27, a CDR2 set forth in SEQ ID NO: 55, and a CDR3 set forth in SEQ ID NO: 94;
wherein, any one of the amino acid sequences described above further comprises a derived sequence optionally subjected to addition, deletion, modification, and/or substitution of at least one amino acid residue and capable of retaining the binding affinity for Trop2.

In another preferred embodiment, the heavy chain variable region has an amino acid sequence set forth in any one of SEQ ID NOs: 192-220.

In another preferred embodiment, the antibody or the antigen-binding protein further comprises a heavy chain comprising a heavy chain constant region.

In another preferred embodiment, the heavy chain constant region is a human heavy chain constant region.

In another preferred embodiment, the heavy chain constant region is a heavy chain constant region Fc domain of a human antibody; and the heavy chain constant region Fc domain of the human antibody comprises a heavy chain constant region Fc domain of human IgG1, IgG2, IgG3, or IgG4.

In another preferred embodiment, the heavy chain constant region comprises an amino acid sequence set forth in SEQ ID NO: 292.

In another preferred embodiment, the heavy chain of the antibody or the antigen-binding protein has an amino acid sequence set forth in any one of SEQ ID NOs: 241-269.

In another preferred embodiment, the antibody or the antigen-binding protein further comprises a light chain, the light chain comprising a light chain variable region having combination of complementarity determining regions (CDRs) selected from the group consisting of:
(1) a CDR1 set forth in SEQ ID NO: 115, a CDR2 set forth in SEQ ID NO: 144, and a CDR3 set forth in SEQ ID NO: 169;
(2) a CDR1 set forth in SEQ ID NO: 116, a CDR2 set forth in SEQ ID NO: 145, and a CDR3 set forth in SEQ ID NO: 170;
(3) a CDR1 set forth in SEQ ID NO: 117, a CDR2 set forth in SEQ ID NO: 146, and a CDR3 set forth in SEQ ID NO: 171;
(4) a CDR1 set forth in SEQ ID NO: 118, a CDR2 set forth in SEQ ID NO: 147, and a CDR3 set forth in SEQ ID NO: 172;
(5) a CDR1 set forth in SEQ ID NO: 119, a CDR2 set forth in SEQ ID NO: 148, and a CDR3 set forth in SEQ ID NO: 173;
(6) a CDR1 set forth in SEQ ID NO: 120, a CDR2 set forth in SEQ ID NO: 146, and a CDR3 set forth in SEQ ID NO: 174;
(7) a CDR1 set forth in SEQ ID NO: 121, a CDR2 set forth in SEQ ID NO: 145, and a CDR3 set forth in SEQ ID NO: 175;
(8) a CDR1 set forth in SEQ ID NO: 122, a CDR2 set forth in SEQ ID NO: 149, and a CDR3 set forth in SEQ ID NO: 176;
(9) a CDR1 set forth in SEQ ID NO: 123, a CDR2 set forth in SEQ ID NO: 145, and a CDR3 set forth in SEQ ID NO: 177;
(10) a CDR1 set forth in SEQ ID NO: 120, a CDR2 set forth in SEQ ID NO: 146, and a CDR3 set forth in SEQ ID NO: 174;
(11) a CDR1 set forth in SEQ ID NO: 124, a CDR2 set forth in SEQ ID NO: 149, and a CDR3 set forth in SEQ ID NO: 178;
(12) a CDR1 set forth in SEQ ID NO: 125, a CDR2 set forth in SEQ ID NO: 148, and a CDR3 set forth in SEQ ID NO: 179;
(13) a CDR1 set forth in SEQ ID NO: 126, a CDR2 set forth in SEQ ID NO: 150, and a CDR3 set forth in SEQ ID NO: 180;
(14) a CDR1 set forth in SEQ ID NO: 127, a CDR2 set forth in SEQ ID NO: 151, and a CDR3 set forth in SEQ ID NO: 180;
(15) a CDR1 set forth in SEQ ID NO: 126, a CDR2 set forth in SEQ ID NO: 150, and a CDR3 set forth in SEQ ID NO: 180;
(16) a CDR1 set forth in SEQ ID NO: 128, a CDR2 set forth in SEQ ID NO: 152, and a CDR3 set forth in SEQ ID NO: 181;
(17) a CDR1 set forth in SEQ ID NO: 127, a CDR2 set forth in SEQ ID NO: 151, and a CDR3 set forth in SEQ ID NO: 180;
(18) a CDR1 set forth in SEQ ID NO: 127, a CDR2 set forth in SEQ ID NO: 151, and a CDR3 set forth in SEQ ID NO: 180;
(19) a CDR1 set forth in SEQ ID NO: 127, a CDR2 set forth in SEQ ID NO: 150, and a CDR3 set forth in SEQ ID NO: 180;
(20) a CDR1 set forth in SEQ ID NO: 127, a CDR2 set forth in SEQ ID NO: 150, and a CDR3 set forth in SEQ ID NO: 180;
(21) a CDR1 set forth in SEQ ID NO: 127, a CDR2 set forth in SEQ ID NO: 150, and a CDR3 set forth in SEQ ID NO: 180;
(22) a CDR1 set forth in SEQ ID NO: 127, a CDR2 set forth in SEQ ID NO: 151, and a CDR3 set forth in SEQ ID NO: 180;
(23) a CDR1 set forth in SEQ ID NO: 129, a CDR2 set forth in SEQ ID NO: 144, and a CDR3 set forth in SEQ ID NO: 182;
(24) a CDR1 set forth in SEQ ID NO: 127, a CDR2 set forth in SEQ ID NO: 151, and a CDR3 set forth in SEQ ID NO: 180;
(25) a CDR1 set forth in SEQ ID NO: 130, a CDR2 set forth in SEQ ID NO: 146, and a CDR3 set forth in SEQ ID NO: 183;
(26) a CDR1 set forth in SEQ ID NO: 127, a CDR2 set forth in SEQ ID NO: 150, and a CDR3 set forth in SEQ ID NO: 180;
(27) a CDR1 set forth in SEQ ID NO: 131, a CDR2 set forth in SEQ ID NO: 153, and a CDR3 set forth in SEQ ID NO: 184;
(28) a CDR1 set forth in SEQ ID NO: 132, a CDR2 set forth in SEQ ID NO: 154, and a CDR3 set forth in SEQ ID NO: 185;
(29) a CDR1 set forth in SEQ ID NO: 133, a CDR2 set forth in SEQ ID NO: 153, and a CDR3 set forth in SEQ ID NO: 184;
(30) a CDR1 set forth in SEQ ID NO: 127, a CDR2 set forth in SEQ ID NO: 150, and a CDR3 set forth in SEQ ID NO: 180;
wherein, any one of the amino acid sequences described above further comprises a derived sequence optionally subjected to addition, deletion, modification, and/or substitution of at least one amino acid residue and capable of retaining the binding affinity for Trop2.

In another preferred embodiment, the light chain variable region has an amino acid sequence set forth in any one of SEQ ID NOs: 221-240.

In another preferred embodiment, the light chain of the antibody or the antigen-binding protein further comprises a light chain constant region.

In another preferred embodiment, the light chain constant region is a human antibody constant region.

In another preferred embodiment, the light chain constant region is a human antibody light chain κ constant region.

In another preferred embodiment, the light chain constant region comprises an amino acid sequence of SEQ ID NO: 293.

In another preferred embodiment, the light chain has an amino acid sequence set forth in any one of SEQ ID NOs: 270-289.

In another preferred embodiment, the antibody or the antigen-binding protein further comprises a derived sequence of any one of the amino acid sequences described above optionally subjected to addition, deletion, modification, and/or substitution of at least one amino acid and capable of retaining the binding affinity for Trop2.

In another preferred embodiment, the affinity F1 of the antibody having the derived sequence for binding to TROP2 and the affinity F0 of a corresponding non-derived antibody for binding to TROP2 are in a ratio (F1/F0) of 0.5-2, preferably 0.7-1.5, and more preferably 0.8-1.2.

In another preferred embodiment, the number of the amino acids being added, deleted, modified, and/or substituted is 1-5 (e.g., 1-3, preferably 1-2, and more preferably 1).

In another preferred embodiment, the derived sequence subjected to addition, deletion, modification, and/or substitution of at least one amino acid and capable of retaining the binding affinity for TROP2 is an amino acid sequence with homology or at least 96% sequence identity.

In another preferred embodiment, the heavy chain variable region of the antibody further comprises a human framework region, and/or the light chain variable region of the antibody further comprises a human framework region.

In another preferred embodiment, the antibody is selected from the group consisting of: an animal-derived antibody, a chimeric antibody, a humanized antibody, a fully human antibody, and a combination thereof.

In another preferred embodiment, the immunogenicity Z1 of the chimeric antibody in human and the immunogenicity Z0 of a non-chimeric antibody (e.g., a murine antibody) in human are in a ratio (Z1/Z0) of 0-0.5, preferably 0-0.2, and more preferably 0-0.05 (e.g., 0.001-0.05).

In another preferred embodiment, the antibody is a fully human monoclonal antibody.

In another preferred embodiment, the antibody is a double-chain antibody.

In another preferred embodiment, the antibody is an antibody full-length protein or an antigen-binding fragment.

In another preferred embodiment, the antibody is in the form of an antibody-drug conjugate.

In another preferred embodiment, the antibody has one or more properties selected from the group consisting of:
(a) capable of binding to HEK293 cells overexpressing human Trop2 in a FACS assay;
(b) capable of binding to CHO-K1 cells overexpressing monkey Trop2 in a FACS assay;
(c) capable of binding to a mouse Trop2 protein in an ELISA assay;
(d) capable of being internalized into HEK293 cells or BxPC-3 cells overexpressing human Trop2;
(e) having ADCC activity against BxPc-3 cells; and/or
(f) having ADCP activity against BxPc-3 cells.

In another preferred embodiment, the antibody is one of antibodies having combinations of heavy chain VH-CDR1, VH-CDR2, and VH-CDR3, and light chain VL-CDR1, VL-CDR2, and VL-CDR3 as shown in Table A:

**Table A**

| **Antibody No.** | **Name of antibody** | **LCDR1** | **LCDR2** | **LCDR3** | **HCDR1** | **HCDR2** | **HCDR3** |
|---|---|---|---|---|---|---|---|
| (1) | PR001128 | 115 | 144 | 169 | 19 | 47 | 86 |
| (2) | PR001130 | 116 | 145 | 170 | 20 | 48 | 87 |
| (3) | PR001131 | 117 | 146 | 171 | 20 | 48 | 87 |
| (4) | PR001132 | 118 | 147 | 172 | 21 | 49 | 88 |
| (5) | PR001133 | 119 | 148 | 173 | 21 | 50 | 89 |
| (6) | PR001134 | 120 | 146 | 174 | 22 | 51 | 90 |
| (7) | PR001138 | 121 | 145 | 175 | 23 | 52 | 91 |
| (8) | PR001139 | 122 | 149 | 176 | 24 | 53 | 92 |
| (9) | PR001142 | 123 | 145 | 177 | 25 | 54 | 87 |
| (10) | PR001143 | 120 | 146 | 174 | 26 | 51 | 93 |
| (11) | PR001145 | 124 | 149 | 178 | 24 | 53 | 92 |
| (12) | PR001147 | 125 | 148 | 179 | 21 | 50 | 89 |
| (13) | PR001150 | 126 | 150 | 180 | 27 | 55 | 94 |
| (14) | PR001151 | 127 | 151 | 180 | 27 | 55 | 95 |
| (15) | PR001152 | 126 | 150 | 180 | 27 | 55 | 94 |
| (16) | PR001153 | 128 | 152 | 181 | 19 | 52 | 96 |
| (17) | PR001154 | 127 | 151 | 180 | 27 | 55 | 95 |
| (18) | PR001155 | 127 | 151 | 180 | 27 | 55 | 95 |
| (19) | PR001156 | 127 | 150 | 180 | 27 | 55 | 97 |
| (20) | PR001158 | 127 | 150 | 180 | 27 | 55 | 94 |
| (21) | PR001159 | 127 | 150 | 180 | 27 | 55 | 94 |
| (22) | PR001160 | 127 | 151 | 180 | 27 | 56 | 95 |
| (23) | PR001162 | 129 | 144 | 182 | 28 | 57 | 98 |
| (24) | PR001163 | 127 | 151 | 180 | 27 | 55 | 95 |
| (25) | PR001164 | 130 | 146 | 183 | 29 | 58 | 99 |
| (26) | PR001165 | 127 | 150 | 180 | 27 | 55 | 94 |
| (27) | PR001166 | 131 | 153 | 184 | 23 | 59 | 100 |
| (28) | PR001168 | 132 | 154 | 185 | 30 | 51 | 101 |
| (29) | PR001170 | 133 | 153 | 184 | 31 | 59 | 100 |
| (30) | PR001171 | 127 | 150 | 180 | 27 | 55 | 94 |

wherein, any one of the amino acid sequences described above further comprises a derived sequence optionally subjected to addition, deletion, modification, and/or substitution of at least one amino acid and capable of retaining the binding affinity for TROP2.

In another preferred embodiment, the antibody is an antibody in Table A having a number selected from the group consisting of: (1), (2), (5), (8), (9), (23), (26), and (27) (corresponding to antibody numbers PR001128, PR001130, PR001133, PR001139, PR001142, PR001162, PR001165, and PR001166 in examples, respectively).

In another preferred embodiment, the antibody is an antibody in Table A having a number selected from the group consisting of: (9), (23), (26), and (27) (corresponding to antibody numbers PR001142, PR001162, PR001165, and PR001166 in examples, respectively).

In another preferred embodiment, the antibody is an antibody in Table A having a number of (27) (corresponding to an antibody number PR001 166 in examples).

In another preferred embodiment, the heavy chain variable region has an amino acid sequence set forth in any one of SEQ ID NOs: 192-220, or an amino acid sequence having at least 80%, 85%, 90%, 91%, 92%, 93%, 94%, 95%, 96%, 97%, 98%, or 99% sequence homology thereto; and/or the light chain variable region has an amino acid sequence set forth in any one of SEQ ID Nos: 221-240, or an amino acid sequence having at least 80%, 85%, 90%, 91%, 92%, 93%, 94%, 95%, 96%, 97%, 98%, or 99% sequence homology thereto.

In another preferred embodiment, the antibody is one of antibodies having combinations of heavy chain variable regions and light chain variable regions as shown in Table B:

**Table B**

| **Antibody No.** | **Name of antibody** | **VL** | **VH** |
|---|---|---|---|
| (1) | PR001128 | 221 | 192 |
| (2) | PR001130 | 222 | 193 |
| (3) | PR001131 | 223 | 193 |
| (4) | PR001132 | 224 | 194 |
| (5) | PR001133 | 225 | 195 |
| (6) | PR001134 | 226 | 196 |
| (7) | PR001138 | 227 | 197 |
| (8) | PR001139 | 228 | 198 |
| (9) | PR001142 | 229 | 199 |
| (10) | PR001143 | 226 | 200 |
| (11) | PR001145 | 230 | 201 |
| (12) | PR001147 | 231 | 202 |
| (13) | PR001150 | 232 | 203 |
| (14) | PR001151 | 233 | 204 |
| (15) | PR001152 | 232 | 205 |
| (16) | PR001153 | 234 | 206 |
| (17) | PR001154 | 233 | 207 |
| (18) | PR001155 | 233 | 208 |
| (19) | PR001156 | 235 | 209 |
| (20) | PR001158 | 235 | 210 |
| (21) | PR001159 | 235 | 211 |
| (22) | PR001160 | 233 | 212 |
| (23) | PR001162 | 236 | 213 |
| (24) | PR001163 | 233 | 214 |
| (25) | PR001164 | 237 | 215 |
| (26) | PR001165 | 235 | 216 |
| (27) | PR001166 | 238 | 217 |
| (28) | PR001168 | 239 | 218 |
| (29) | PR001170 | 240 | 219 |
| (30) | PR001171 | 235 | 220 |

In another preferred embodiment, the antibody is an antibody in Table B having a number selected from the group consisting of: (1), (2), (5), (8), (9), (23), (26), and (27) (corresponding to antibody numbers PR001128, PR001130, PR001133, PR001139, PR001142, PR001162, PR001165, and PR001166 in examples, respectively).

In another preferred embodiment, the antibody is an antibody in Table B having a number selected from the group consisting of: (9), (23), (26), and (27) (corresponding to antibody numbers PR001142, PR001162, PR001165, and PR001166 in examples, respectively).

In another preferred embodiment, the antibody is an antibody in Table B having a number of (27) (corresponding to an antibody number PR001 166 in examples).

In another preferred embodiment, the antibody is one of antibodies having combinations of heavy chains and light chains as shown in Table C:

**Table C**

| **Antibody No.** | **Name of antibody** | **LC** | **HC** |
|---|---|---|---|
| (1) | PR001128 | 270 | 241 |
| (2) | PR001130 | 271 | 242 |
| (3) | PR001131 | 272 | 242 |
| (4) | PR001132 | 273 | 243 |
| (5) | PR001133 | 274 | 244 |
| (6) | PR001134 | 275 | 245 |
| (7) | PR001138 | 276 | 246 |
| (8) | PR001139 | 277 | 247 |
| (9) | PR001142 | 278 | 248 |
| (10) | PR001143 | 275 | 249 |
| (11) | PR001145 | 279 | 250 |
| (12) | PR001147 | 280 | 251 |
| (13) | PR001150 | 281 | 252 |
| (14) | PR001151 | 282 | 253 |
| (15) | PR001152 | 281 | 254 |
| (16) | PR001153 | 283 | 255 |
| (17) | PR001154 | 282 | 256 |
| (18) | PR001155 | 282 | 257 |
| (19) | PR001156 | 284 | 258 |
| (20) | PR001158 | 284 | 259 |
| (21) | PR001159 | 284 | 260 |
| (22) | PR001160 | 282 | 261 |
| (23) | PR001162 | 285 | 262 |
| (24) | PR001163 | 282 | 263 |
| (25) | PR001164 | 286 | 264 |
| (26) | PR001165 | 284 | 265 |
| (27) | PR001166 | 287 | 266 |
| (28) | PR001168 | 288 | 267 |
| (29) | PR001170 | 289 | 268 |
| (30) | PR001171 | 284 | 269 |

In another preferred embodiment, the antibody is an antibody in Table C having a number selected from the group consisting of: (1), (2), (5), (8), (9), (23), (26), and (27)(corresponding to antibody numbers PR001128, PR001130, PR001133, PR001139, PR001142, PR001162, PR001165, and PR001166 in examples, respectively).

In another preferred embodiment, the antibody is an antibody in Table C having a number selected from the group consisting of: (9), (23), (26), and (27)(corresponding to antibody numbers PR001142, PR001162, PR001165, and PR001166 in examples, respectively).

In another preferred embodiment, the antibody is an antibody in Table C having a number of (27) (corresponding to an antibody number PR001 166 in examples).

In a second aspect of the present invention, provided is a recombinant protein comprising:
(i) the antibody or the antigen-binding protein according to the first aspect of the present invention; and
(ii) an optional tag sequence to assist in expression and/or purification.

In another preferred embodiment, the tag sequence comprises a 6His tag.

In another preferred embodiment, the recombinant protein (or polypeptide) comprises a fusion protein.

In another preferred embodiment, the recombinant protein is a monomer, dimer, or polymer.

In a third aspect of the present invention, provided is a polynucleotide encoding a polypeptide selected from the group consisting of: the antibody or the antigen-binding protein according to the first aspect of the present invention, and/or the recombinant protein according to the second aspect of the present invention.

In a fourth aspect of the present invention, provided is a vector comprising the polynucleotide according to the third aspect of the present invention.

In another preferred embodiment, the vector includes: bacterial plasmids, bacteriophages, yeast plasmids, plant cell viruses, mammalian cell viruses such as adenoviruses and retroviruses, or other vectors.

In another preferred embodiment, the vector is an oncolytic viral vector.

In a fifth aspect of the present invention, provided is a genetically engineered host cell comprising the vector according to the fourth aspect of the present invention or having the polynucleotide according to the third aspect of the present invention integrated in the genome thereof.

In a sixth aspect of the present invention, provided is a method for preparing the antibody or the antigen-binding fragment thereof according to the first aspect, comprising culturing the host cell according to the fifth aspect under conditions allowing an expression of the antibody or the antigen-binding fragment thereof, and isolating the antibody or the antigen-binding fragment thereof from a cultured host cell culture.

In a seventh aspect of the present invention, provided is an antibody-drug conjugate comprising:
(a) an antibody moiety comprising the antibody or the antigen-binding protein according to the first aspect of the present invention; and
(b) a conjugate moiety conjugated with the antibody moiety, wherein the conjugate moiety is selected from the group consisting of: a detectable label, a drug, a toxin, a cytokine, a radionuclide, an enzyme, and a combination thereof.

In another preferred embodiment, the antibody moiety is conjugated with the conjugate moiety through a chemical bond or a linker.

In another preferred embodiment, the conjugate moiety includes: cytotoxins, alkylating agents, DNA minor groove binding molecules, DNA intercalators, DNA cross-linking agents, histone deacetylase inhibitors, nuclear export inhibitors, proteasome inhibitors, inhibitors of topoisomerase I or II, heat shock protein inhibitors, tyrosine kinase inhibitors, antibiotics and antimitotic agents, preferably SN-38.

In an eighth aspect of the present invention, provided is a chimeric antigen receptor (CAR) comprising the antibody or the antigen-binding protein according to the first aspect of the present invention.

In a ninth aspect of the present invention, provided is an immune cell expressing or exposing outside the cell membrane the antibody or the antigen-binding protein according to the first aspect of the present invention. In another preferred embodiment, the immune cell includes NK cells and T cells.

In another preferred embodiment, the immune cell is from a human or a non-human mammal (e.g., a mouse).

In another preferred embodiment, the immune cell includes the chimeric antigen receptor according to the eighth aspect of the present invention, and the immune cell is preferably a chimeric antigen receptor T cell (CAR-T cell) or a chimeric antigen receptor NK cell (CAR-NK cell).

In a tenth aspect of the present invention, provided is a multispecific antibody comprising the Trop-2-binding antibody or the antigen-binding fragment according to the first aspect of the present invention, and an additional antibody or a fragment or an antibody analog thereof.

In another preferred embodiment, the multispecific antibody is formed by conjugating a first antibody or an antigen-binding fragment thereof with other antibodies or antigen-binding fragments or antibody analogs thereof, wherein each of the antibodies or the antigen-binding fragments or antibody analogs thereof retain its original binding specificity, and the first antibody or the antigen-binding fragment thereof is the antibody or the antigen-binding fragment thereof according to the present invention.

In another preferred embodiment, the multispecific antibody is a bispecific antibody or a trispecific antibody or a tetraspecific antibody.

In an eleventh aspect of the present invention, provided is a pharmaceutical composition comprising:
(i) an active ingredient selected from the group consisting of: the antibody or the antigen-binding protein according to the first aspect of the present invention, the recombinant protein according to the second aspect of the present invention, the antibody-drug conjugate according to the seventh aspect of the present invention, the immune cell according to the ninth aspect of the present invention, the multispecific antibody according to the tenth aspect of the present invention, and a combination thereof; and
(ii) a pharmaceutically acceptable carrier.

In another preferred embodiment, the pharmaceutical composition is a liquid formulation. In another preferred embodiment, the pharmaceutical composition is an injection.

In another preferred embodiment, the pharmaceutical composition comprises 0.01%-99.99% of the antibody or the antigen-binding protein according to the first aspect of the present invention, the recombinant protein according to the second aspect of the present invention, the antibody-drug conjugate according to the seventh aspect of the present invention, the immune cell according to the ninth aspect of the present invention, the multispecific antibody according to the tenth aspect of the present invention, or a combination thereof, and 0.01%-99.99% of a pharmaceutically acceptable carrier, the percentage being a mass percentage of the pharmaceutical composition.

In another preferred embodiment, the pharmaceutical composition further comprises a second therapeutic agent.

In another preferred embodiment, the second therapeutic agent comprises a second antibody or a chemotherapeutic agent.

In another preferred example, the second antibody includes: an LAG-3 antibody, a PD-1 antibody, a CTLA-4 antibody, or the like.

In another preferred embodiment, the chemotherapeutic agent is selected from the group consisting of: docetaxel, carboplatin, capecitabine, vinorelbine, and a combination thereof. In another preferred embodiment, the chemotherapeutic agent is a cytotoxic agent including: SN-38, epirubicin, oxaliplatin, or 5-FU.

In a twelfth aspect of the present invention, provided is a method for treating, preventing, or ameliorating a tumor, which comprises administering to a subject in need thereof the antibody or the antigen-binding protein according to the first aspect of the present invention, the recombinant protein according to the second aspect, the antibody-drug conjugate according to the seventh aspect, the immune cell according to the ninth aspect, the multispecific antibody according to the tenth aspect, and the pharmaceutical composition according to the eleventh aspect.

In another preferred embodiment, the tumor may be a solid tumor or a non-solid tumor.

In another preferred embodiment, the solid tumor is selected from the group consisting of: breast cancer, gastric cancer, pancreatic cancer, ovarian cancer, intestinal cancer, lung cancer, cervical cancer, and other tumors positive for Trop2 expression.

In another preferred embodiment, the method further comprises administration of a second therapeutic agent.

In another preferred embodiment, the second therapeutic agent comprises a second antibody or a chemotherapeutic agent.

In another preferred example, the second antibody includes: an LAG-3 antibody, a PD-1 antibody, or a CTLA-4 antibody.

In another preferred embodiment, the chemotherapeutic agent is selected from the group consisting of: docetaxel, carboplatin, capecitabine, vinorelbine, and a combination thereof. In another preferred embodiment, the chemotherapeutic agent is a cytotoxic agent including: SN-38, epirubicin, oxaliplatin, or 5-FU.

In a thirteenth aspect of the present invention, provided is use of an active ingredient selected from the group consisting of: the antibody or the antigen-binding protein according to the first aspect of the present invention, the recombinant protein according to the second aspect of the present invention, the antibody-drug conjugate according to the seventh aspect of the present invention, the immune cell according to the ninth aspect of the present invention, the multispecific antibody according to the tenth aspect of the present invention, and a combination thereof, wherein the active ingredient is used for: (a) preparing a diagnostic reagent or kit for a disease related to abnormal expression of TROP2; and/or (b) preparing a medicament for preventing and/or treating a disease related to abnormal expression of TROP2.

In another preferred embodiment, the diagnostic reagent is an assay strip or an assay plate. In another preferred embodiment, the disease related to abnormal expression or function of TROP2 primarily includes a tumor.

In another preferred embodiment, the tumor may be a solid tumor or a non-solid tumor.

In another preferred embodiment, the solid tumor is selected from the group consisting of: breast cancer, gastric cancer, pancreatic cancer, ovarian cancer, intestinal cancer, lung cancer, cervical cancer, and other tumors positive for Trop2 expression.

In another preferred embodiment, the diagnostic reagent or kit is used for:
(1) detecting a TROP2 protein in a sample;
(2) detecting an endogenous TROP2 protein in cells; and/or
(3) detecting cells expressing a TROP2 protein.

In another preferred embodiment, the antibody is in the form of an antibody-drug conjugate (ADC).

In a fourteenth aspect of the present invention, provided is a method for *in vitro* detection (including diagnostic or non-diagnostic detection) of a TROP2 protein in a sample, comprising the steps of:
(1) under conditions allowing formation of a complex by the antibody or the antigen-binding fragment according to the first aspect of the present invention and Trop-2, contacting the sample with the antibody or the antigen-binding protein; and
(2) detecting formation of an antigen-antibody complex, wherein the formation of the complex is indicative of the presence of the TROP2 protein in the sample.

In another preferred embodiment, the detection is for a non-diagnostic purpose. In another preferred embodiment, the non-diagnostic purpose includes using the antibody or the antigen-binding protein according to the first aspect for pathway studies, drug screening, histochemical assays, and the like.

In a fifteenth aspect of the present invention, provided is an assay plate comprising: a substrate (support plate) and a test strip comprising the antibody or the antigen-binding protein according to the first aspect of the present invention, the recombinant protein according to the second aspect of the present invention, the antibody-drug conjugate according to the seventh aspect of the present invention, the immune cell according to the ninth aspect of the present invention, or a combination thereof.

In a sixteenth aspect of the present invention, provided is a kit comprising:
(1) a first container comprising the antibody or the antigen-binding fragment of the present invention; and optionally
(2) a second container comprising a secondary antibody against the antibody of the present invention;

In another preferred embodiment, the kit comprises the assay plate according to the fifteenth aspect of the present invention.

In another preferred embodiment, the kit further comprises instructions for use.

In a seventeenth aspect of the present invention, provided is an administration device comprising:
(i) an infusion module for administering to a subject a pharmaceutical composition comprising an active ingredient;
(ii) a pharmaceutical composition for infusion comprising an active ingredient selected from the group consisting of: the antibody or the antigen-binding protein according to the first aspect of the present invention, the recombinant protein according to the second aspect of the present invention, the antibody-drug conjugate according to the seventh aspect of the present invention, the immune cell according to the ninth aspect of the present invention, and a combination thereof; and
(iii) an optional pharmacodynamic monitoring module.

In another preferred embodiment, the administration includes parenteral administration and non-parenteral administration.

In another preferred embodiment, the parenteral administration includes injection administration, and the route of injection used includes: intravenous, intramuscular, intra-arterial, intramembranous, intracapsular, intraorbital, intracardiac, intradermal, intraperitoneal, transtracheal, subcutaneous, subcuticular, intra-articular, subcapsular, subarachnoid, intraspinal, supradural and intrasternal injection and bolus.

In another preferred embodiment, the non-parenteral administration includes topical, epidermal, or mucosal administration, e.g., intranasal, oral, vaginal, rectal, sublingual, or topical administration.

In another preferred embodiment, the infusion module is a needleless hypodermic device, a micro-infusion pump, a transdermal administration device, a bolus injection device, or an osmotic device.

It should be understood that within the scope of the present invention, the above various technical features of the present invention and those specifically described below (e.g., in the examples) may be combined with each other to form new or preferred technical solutions. Due to the length limit, they will not be described herein one by one.

### BRIEF DESCRIPTION OF THE DRAWINGS

FIG. 1 shows the binding ability of the antibodies of the present invention to 293T-human Trop2 cells.
FIG. 2 shows the internalizing killing activity of the antibodies of the present invention against BxPC-3 tumor cells after conjugation with MMAF.
FIG. 3 shows the binding ability of the antibodies of the present invention to tumor cells BxPC-3.
FIG. 4 shows the binding ability of the antibodies of the present invention to CHO-K1 cells overexpressing monkey Trop2.
FIG. 5 shows the binding ability of the antibodies of the present invention to a mouse Trop2 protein.
FIG. 6 shows the ADCC activity mediated by the antibodies of the present invention.
FIG. 7 shows the ADCP activity of the antibodies of the present invention.
FIG. 8 shows the CDC activity of the antibodies of the present invention.
FIG. 9 shows the endocytosis of the antibodies of the present invention on HEK293/hTrop2 cells at 4 °C and 37 °C.
FIG. 10 shows the endocytosis of the antibodies of the present invention on tumor cells BxPC-3 at 4 °C and 37 °C.

### DETAILED DESCRIPTION

After an extensive and intensive research and extensive screening, the inventor developed a Trop2-targeted binding protein and use thereof for the first time. On this basis, the present invention is completed.

The present invention aims to solve the technical problems of lack of anti-tumor efficacy, poor safety and the like of antibody drugs in the prior art, and provides a Trop2-targeted binding protein and use thereof.

For a better understanding of the present invention, some terms are first defined. Other definitions are set forth throughout the detailed description.

The term "Trop2" refers to trophoblast cell-surface antigen 2. This term includes variants, homologs, analogs, orthologs, and/or paralogs. For example, an antibody specific for human Trop2 may, in certain circumstances, cross-react with a Trop2 protein of another species, e.g., monkey. In other embodiments, an antibody specific for a human Trop2 protein may be completely specific for the human Trop2 protein without cross-reacting with proteins of other species or other types, or may cross-react with Trop2 proteins of some other species rather than all other species.

The term "human Trop2" refers to a Trop2 protein having a human amino acid sequence, for example, an amino acid sequence under the Genbank accession No. NP_002344.2.

The term "monkey Trop2" refers to a Trop2 protein having a monkey amino acid sequence, for example, an amino acid sequence under the Genbank accession No. XP_005543292.1. The term "mouse Trop2" refers to a Trop2 protein having a mouse amino acid sequence, for example, an amino acid sequence under the Genbank accession No. NP_064431.2.

The term "antibody" herein is intended to include full-length antibodies and any antigen-binding fragment (i.e., antigen-binding moiety) or single chain thereof. The full-length antibody is a glycoprotein comprising at least two heavy (H) chains and two light (L) chains, the heavy chain and the light chain being linked by disulfide bonds. Each heavy chain is composed of a heavy chain variable region (abbreviated as V_{H}) and a heavy chain constant region. The heavy chain constant region is composed of three domains, C_{H1}, C_{H2}, and C_{H3}. Each light chain is composed of a light chain variable region (abbreviated as V_{L}) and a light chain constant region. The light chain constant region is composed of one domain C_{L}. The V_{H} and V_{L} regions can also be divided into hypervariable regions, referred to as complementarity determining regions (CDRs), which are separated by conserved framework regions (FRs). Each V_{H} or V_{L} is composed of three CDRs and four FRs arranged from the amino terminus to the carboxyl terminus in the order of FR1, CDR1, FR2, CDR2, FR3, CDR3, and FR4. The variable regions of the heavy and light chains contain domains that interact with antigens. The constant regions of the antibody can mediate the binding of immunoglobulins to host tissues or factors, including the binding of various cells of the immune system (e.g., effector cells) to the first component (C1q) of the classical complement system.

The term "antigen-binding moiety" of an antibody (or abbreviated as an antibody moiety) herein refers to one or more fragments of an antibody that retain the ability to specifically bind to an antigen (e.g., a Trop2 protein). It has been demonstrated that the antigen-binding function of an antibody can be performed by fragments of a full-length antibody. Examples of binding fragments encompassed within the "antigen-binding moiety" of an antibody include (i) a Fab fragment, a monovalent fragment consisting of V_{L}, V_{H}, C_{L}, and C_{H1}; (ii) a F(ab')₂ fragment, a bivalent fragment comprising two Fab fragments linked by a disulfide bridge in the hinge region; (iii) an Fd fragment consisting of V_{H} and C_{H1}; (iv) an Fv fragment consisting of V_{L} and V_{H} of a single arm of an antibody; (v) a dAb fragment consisting of V_{H}; (vi) an isolated complementarity determining region (CDR); and (vii) a nanobody, a heavy chain variable region comprising a single variable domain and two constant domains. Furthermore, although the two domains, V_{L} and V_{H}, of an Fv fragment are encoded by different genes, they can be linked by recombinant methods through a synthetic linker that brings the two into a single protein chain, in which the V_{L} and V_{H} regions are paired to form a monovalent molecule (known as single chain Fc (scFv)). These single chain antibodies are also intended to be included within the meaning of the term. These antibody fragments can be obtained by conventional techniques known to those skilled in the art, and the fragments can be functionally screened in the same manner as for intact antibodies.

The term "isolated antibody" as used herein refers to an antibody that is substantially free of other antibodies having different antigenic specificities. For example, an isolated antibody that specifically binds to a Trop2 protein is substantially free of antibodies that specifically bind to antigens other than the Trop2 protein. However, an isolated antibody that specifically binds to a human Trop2 protein may have cross-binding activity against other antigens, such as Trop2 proteins of other species. Furthermore, an isolated antibody is substantially free of other cellular materials and/or chemicals.

The term "monoclonal antibody" or "mAb" or "monoclonal antibody composition" refers to an antibody molecule product consisting of a single molecule. The monoclonal antibody composition exhibits single binding specificity and affinity for a particular epitope.

The term "human antibody" refers to an antibody in which variable region frameworks and CDR regions are derived from human germline immunoglobulin sequences. Furthermore, if the antibody comprises a constant region, the constant region is also derived from human germline immunoglobulin sequences. The human antibody of the present invention may comprise amino acid residues not encoded by human germline immunoglobulin sequences, e.g., mutations introduced by *in vitro* random or point mutations or by *in vivo* somatic mutations. However, the term "human antibody" does not include antibodies in which CDR sequences derived from other mammalian species are inserted into human framework sequences.

The terms "antibody recognizing an antigen" and "antibody specific for an antigen" are used interchangeably herein with the term "antibody specifically binding to an antigen".

Herein, an antibody that "specifically binds to human Trop2" refers to an antibody that binds to human Trop2 (and possibly also to Trop2 of other non-human species) but does not substantially bind to non-Trop2 proteins. Preferably, the antibody binds to a human Trop2 protein with "high affinity", i.e., with a K_{D} value of 1.0×10⁻⁸ M or less, more preferably 5.0×10⁻⁹ M or less.

The term "not substantially bind" to a protein or cell refers to not binding to the protein or cell or not binding to it with high affinity, i.e., the binding protein or cell has a K_{D} of 1.0×10⁻⁶ M or greater, preferably 1.0×10⁻⁵ M or greater, more preferably 1.0×10⁻⁴ M or greater or 1.0×10⁻³ M or greater, and more preferably 1.0×10⁻² M or greater.

The term "high affinity" for IgG antibodies means that K_{D} for an antigen is 1.0×10⁻⁶ M or less, preferably 5.0×10⁻⁸ M or less, more preferably 1.0×10⁻⁸ M or less or 5.0×10⁻⁹ M or less, and more preferably 1.0×10⁻⁹ M or less. For other antibody subtypes, "high affinity" binding may vary. For example, a "high affinity" binding of an IgM subtype means that K_{D} is 10⁻⁶ M or less, preferably 10⁻⁷ M or less, and more preferably 10⁻⁸ M or less.

The term "K_{assoc}" or "Kₐ" refers to the association rate of a particular antibody-antigen interaction, while the term "K_{dis}" or "K_{d}" refers to the dissociation rate of a particular antibody-antigen interaction. The term "K_{D}" refers to a dissociation constant, which is derived from the ratio of K_{d} to Kₐ (K_{d}/Kₐ), and is expressed in molar concentration (M). The K_{D} value of an antibody can be determined by methods known in the art. A preferred method for determining the K_{D} of an antibody is by using a surface plasmon resonance instrument (SPR), preferably by using a biosensing system such as the Biacore^{™} or Octet Red96e system.

The term "EC₅₀", also known as half maximal effect concentration, refers to an antibody concentration that causes 50% of the maximal effect.

The term "antibody-dependent cellular cytotoxicity", "antibody-dependent cell-mediated cytotoxicity", or "ADCC" refers to cell-mediated immune defense in which effector cells of the immune system actively lyse target cells, e.g., cancer cells, with a cell membrane surface antigen binding to an antibody, e.g., a Trop2 antibody.

The term "antibody-dependent cell-mediated phagocytosis" or "ADCP" refers to such an effect that after the binding of an antibody to a corresponding antigen on a target cell, an Fc fragment of the antibody binds to an Fc receptor on an effector cell, such as a phagocytic cell, thereby inducing phagocytosis of the target cell by the effector cell.

The term "subject" includes any human or non-human animal. The term "non-human animal" includes all vertebrates, e.g., mammals and non-mammals, e.g., non-human primates, sheep, dogs, cats, cattle, horses, chickens, amphibians, and reptiles, preferably mammals, e.g., non-human primates, sheep, dogs, cats, cattle, and horses.

The term "therapeutically effective amount" refers to an amount of the antibodies of the present invention sufficient to prevent or alleviate symptoms related to a disease or disorder (e.g., cancer). The therapeutically effective amount is related to the disease to be treated, wherein the actual effective amount can be readily determined by those skilled in the art.

Aspects of the present invention are described in more detail below.

Binding Specificity of Trop2 Antibodies to Trop2 and Other Beneficial Functional

### Characteristics

The antibodies of the present invention specifically bind to human or monkey Trop2, and also bind to mouse Trop2. Specifically, the antibodies of the present invention bind to human or monkey Trop2 with an EC₅₀ value comparable to or lower than that for hRS7. Furthermore, the antibodies of the present invention also have internalization activity or ADCC or ADCP activity comparable to or better than that of hRS7.

The preferred antibody of the present invention is a monoclonal antibody. Furthermore, the antibody may be, for example, a murine, chimeric or human monoclonal antibody, preferably a human antibody.

### Trop2 Monoclonal Antibody

The preferred antibody of the present invention is a monoclonal antibody with structural and chemical properties described below. V_{H} of the Trop2 antibody comprises any one of amino acid sequences of SEQ ID NOs: 192-220. V_{L} of the Trop2 antibody comprises any one of amino acid sequences of SEQ ID NOs: 221-240. The sequences of heavy/light chain variable regions of the antibodies are listed in Tables 1a and 1b below. The heavy and light chain constant regions of all antibodies comprise amino acid sequences of SEQ ID NOs: 292 and 293, respectively. The antibody may also comprise other suitable sequences of heavy and light chain constant regions.

**Table 1 a. Summary of amino acid sequences of the heavy/light chain variable regions and CDRs (SEQ ID NO)**

| **Antibody No.** | **Light chain** | **Heavy chain** | **VL** | **VH** | **LCDR1** | **LCDR2** | **LCDR3** | **HCDR1** | **HCDR2** | **HCDR3** |
|---|---|---|---|---|---|---|---|---|---|---|
| PR001128 | 270 | 241 | 221 | 192 | 115 | 144 | 169 | 19 | 47 | 86 |
| PR001130 | 271 | 242 | 222 | 193 | 116 | 145 | 170 | 20 | 48 | 87 |
| PR001131 | 272 | 242 | 223 | 193 | 117 | 146 | 171 | 20 | 48 | 87 |
| PR001132 | 273 | 243 | 224 | 194 | 118 | 147 | 172 | 21 | 49 | 88 |
| PR001133 | 274 | 244 | 225 | 195 | 119 | 148 | 173 | 21 | 50 | 89 |
| PR001134 | 275 | 245 | 226 | 196 | 120 | 146 | 174 | 22 | 51 | 90 |
| PR001138 | 276 | 246 | 227 | 197 | 121 | 145 | 175 | 23 | 52 | 91 |
| PR001139 | 277 | 247 | 228 | 198 | 122 | 149 | 176 | 24 | 53 | 92 |
| PR001142 | 278 | 248 | 229 | 199 | 123 | 145 | 177 | 25 | 54 | 87 |
| PR001143 | 275 | 249 | 226 | 200 | 120 | 146 | 174 | 26 | 51 | 93 |
| PR001145 | 279 | 250 | 230 | 201 | 124 | 149 | 178 | 24 | 53 | 92 |
| PR001147 | 280 | 251 | 231 | 202 | 125 | 148 | 179 | 21 | 50 | 89 |
| PR001150 | 281 | 252 | 232 | 203 | 126 | 150 | 180 | 27 | 55 | 94 |
| PR001151 | 282 | 253 | 233 | 204 | 127 | 151 | 180 | 27 | 55 | 95 |
| PR001152 | 281 | 254 | 232 | 205 | 126 | 150 | 180 | 27 | 55 | 94 |
| PR001153 | 283 | 255 | 234 | 206 | 128 | 152 | 181 | 19 | 52 | 96 |
| PR001154 | 282 | 256 | 233 | 207 | 127 | 151 | 180 | 27 | 55 | 95 |
| PR001155 | 282 | 257 | 233 | 208 | 127 | 151 | 180 | 27 | 55 | 95 |
| PR001156 | 284 | 258 | 235 | 209 | 127 | 150 | 180 | 27 | 55 | 97 |
| PR001158 | 284 | 259 | 235 | 210 | 127 | 150 | 180 | 27 | 55 | 94 |
| PR001159 | 284 | 260 | 235 | 211 | 127 | 150 | 180 | 27 | 55 | 94 |
| PR001160 | 282 | 261 | 233 | 212 | 127 | 151 | 180 | 27 | 56 | 95 |
| PR001162 | 285 | 262 | 236 | 213 | 129 | 144 | 182 | 28 | 57 | 98 |
| PR001163 | 282 | 263 | 233 | 214 | 127 | 151 | 180 | 27 | 55 | 95 |
| PR001164 | 286 | 264 | 237 | 215 | 130 | 146 | 183 | 29 | 58 | 99 |
| PR001165 | 284 | 265 | 235 | 216 | 127 | 150 | 180 | 27 | 55 | 94 |
| PR001166 | 287 | 266 | 238 | 217 | 131 | 153 | 184 | 23 | 59 | 100 |
| PR001168 | 288 | 267 | 239 | 218 | 132 | 154 | 185 | 30 | 51 | 101 |
| PR001170 | 289 | 268 | 240 | 219 | 133 | 153 | 184 | 31 | 59 | 100 |
| PR001171 | 284 | 269 | 235 | 220 | 127 | 150 | 180 | 27 | 55 | 94 |
| hRS7 | 291 | 290 | | | | | | | | |

**Table 1b. Summary of amino acid sequences of framework regions of heavy/light chain variable regions (SEQ ID NO)**

| **Antibody No.** | **HFWR1** | **HFWR2** | **HFWR3** | **HFWR4** | **LFWR1** | **LFWR2** | **LFWR3** | **LFWR4** |
|---|---|---|---|---|---|---|---|---|
| PR001128 | 1 | 32 | 60 | 102 | 105 | 134 | 155 | 186 |
| PR001130 | 1 | 32 | 61 | 102 | 106 | 135 | 156 | 187 |
| PR001131 | 1 | 32 | 61 | 102 | 107 | 136 | 157 | 188 |
| PR001132 | 2 | 33 | 62 | 102 | 108 | 135 | 158 | 187 |
| PR001133 | 3 | 34 | 63 | 102 | 109 | 135 | 159 | 187 |
| PR001134 | 4 | 35 | 64 | 103 | 109 | 137 | 160 | 189 |
| PR001138 | 5 | 36 | 65 | 102 | 106 | 138 | 161 | 187 |
| PR001139 | 6 | 37 | 66 | 102 | 110 | 139 | 162 | 187 |
| PR001142 | 1 | 32 | 67 | 102 | 106 | 135 | 163 | 190 |
| PR001143 | 7 | 35 | 68 | 102 | 109 | 137 | 160 | 189 |
| PR001145 | 6 | 38 | 66 | 102 | 111 | 140 | 162 | 187 |
| PR001147 | 3 | 39 | 69 | 102 | 109 | 135 | 158 | 187 |
| PR001150 | 8 | 40 | 70 | 104 | 112 | 141 | 164 | 186 |
| PR001151 | 6 | 35 | 71 | 104 | 112 | 135 | 164 | 186 |
| PR001152 | 9 | 35 | 72 | 104 | 112 | 141 | 164 | 186 |
| PR001153 | 1 | 32 | 73 | 102 | 113 | 142 | 165 | 188 |
| PR001154 | 10 | 35 | 74 | 104 | 112 | 135 | 164 | 186 |
| PR001155 | 11 | 35 | 75 | 104 | 112 | 135 | 164 | 186 |
| PR001156 | 12 | 35 | 76 | 104 | 112 | 135 | 164 | 186 |
| PR001158 | 13 | 35 | 77 | 104 | 112 | 135 | 164 | 186 |
| PR001159 | 10 | 35 | 78 | 104 | 112 | 135 | 164 | 186 |
| PR001160 | 10 | 35 | 79 | 104 | 112 | 135 | 164 | 186 |
| PR001162 | 14 | 41 | 80 | 102 | 105 | 140 | 166 | 186 |
| PR001163 | 15 | 42 | 75 | 104 | 112 | 135 | 164 | 186 |
| PR001164 | 16 | 43 | 81 | 102 | 112 | 135 | 164 | 189 |
| PR001165 | 10 | 35 | 82 | 104 | 112 | 135 | 164 | 186 |
| PR001166 | 17 | 44 | 83 | 102 | 111 | 143 | 167 | 189 |
| PR001168 | 18 | 45 | 84 | 102 | 114 | 140 | 168 | 191 |
| PR001170 | 17 | 46 | 83 | 102 | 111 | 143 | 167 | 189 |
| PR001171 | 6 | 35 | 85 | 104 | 112 | 135 | 164 | 186 |

V_{H} and/or V_{L} sequences (or CDR sequences) of other Trop2 antibodies that bind to human Trop2 may be "mixed and paired" with V_{H} and/or V_{L} sequences (or CDR sequences) of the antibodies of the present invention. Preferably, when V_{H} and V_{L} (or CDRs therein) are mixed and paired, the V_{H} sequence in a particular V_{H}/V_{L} pair may be replaced by a structurally similar V_{H} sequence. Similarly, it is preferred that the V_{L} sequence in a particular V_{H}/V_{L} pair is replaced by a structurally similar V_{L} sequence.

Therefore, in one embodiment, the antibody or the antigen-binding moiety thereof of the present invention comprises:
(a) a heavy chain variable region comprising an amino acid sequence listed in Table 1a; and
(b) a light chain variable region comprising an amino acid sequence listed in Table 1a, or a V_{L} of another Trop2 antibody, wherein the antibody specifically binds to human Trop2.

In another embodiment, the antibody or the antigen-binding moiety thereof of the present invention comprises:
(a) a CDR1, a CDR2, and a CDR3 of a heavy chain variable region listed in Table 1a; and
(b) a CDR1, a CDR2, and a CDR3 of a light chain variable region listed in Table 1a, or CDRs of another Trop2 antibody, wherein the antibody specifically binds to human Trop2. In another embodiment, the antibody or the antigen-binding moiety thereof of the present invention comprises CDRs of a heavy chain variable region of a Trop2 antibody and CDRs of other antibodies that bind to human Trop2, e.g., a CDR1, a CDR2, and/or a CDR3 of the heavy chain variable region, and/or a CDR1, a CDR2, and/or a CDR3 of the light chain variable region of another Trop2 antibody.

Furthermore, it is well known in the art that the CDR3 domain, independent of the CDR1 and/or CDR2, can independently determine the binding specificity of antibodies for the same antigen, and can predict that multiple antibodies with the same binding specificity can be generated based on this CDR3 sequence.

In another embodiment, the antibody of the present invention comprises a CDR2 of a heavy chain variable region of a Trop2 antibody, and at least a CDR3 of heavy and/or light chain variable regions of the Trop2 antibody or a CDR3 of heavy and/or light chain variable regions of another Trop2 antibody, wherein the antibody is capable of specifically binding to human Trop2. Preferably, these antibodies (a) compete for binding to Trop2; (b) retain functional properties; (c) bind to the same epitope; and/or (d) have similar binding affinity to the Trop2 antibody of the present invention. In another embodiment, the antibody may further comprise a CDR2 of a light chain variable region of a Trop2 antibody or a CDR2 of a light chain variable region of another Trop2 antibody, wherein the antibody specifically binds to human Trop2. In another embodiment, the antibody of the present invention may comprise a CDR1 of heavy/light chain variable regions of a Trop2 antibody or a CDR1 of heavy and/or light chain variable regions of another Trop2 antibody, wherein the antibody specifically binds to human Trop2.

### Conservative Modification

In another embodiment, the antibody of the present invention comprises heavy and/or light chain variable region sequences or CDR1, CDR2 and CDR3 sequences with one or more conservative modifications relative to the Trop2 antibody of the present invention. It is known in the art that some conservative sequence modifications do not eliminate the antigen-binding activity. See, for example, Brummell et al., (1993) Biochem 32:1180-8; de Wildt et al., (1997) Prot. Eng. 10:835-41; Komissarov et al., (1997) J. Biol. Chem. 272:26864-26870; Hall et al., (1992) J. Immunol. 149:1605-12; Kelley and O'Connell (1993) Biochem. 32:6862-35; Adib-Conquy et al., (1998) Int. Immunol. 10:341-6, and Beers et al., (2000) Clin. Can. Res. 6:2835-43.

Therefore, in one embodiment, the antibody comprises a heavy chain variable region and/or a light chain variable region each comprising a CDR1, a CDR2 and a CDR3, wherein:
(a) the CDR1 of the heavy chain variable region comprises a sequence listed in Table 1a, and/or a conservative modification thereof; and/or
(b) the CDR2 of the heavy chain variable region comprises a sequence listed in Table 1a, and/or a conservative modification thereof; and/or
(c) the CDR3 of the heavy chain variable region comprises a sequence listed in Table 1a, and/or a conservative modification thereof; and/or
(d) the CDR1 and/or CDR2 and/or CDR3 of the light chain variable region comprise sequences listed in Table 1a, and/or conservative modifications thereof; and
(e) the antibody specifically binds to human Trop2.

The antibody of the present invention has one or more of the following functional characteristics, e.g., high affinity for human Trop2, and the ability to elicit ADCC or CDC for a Trop2 expressing cell.

In multiple embodiments, the antibody may be, for example, a murine, human, chimeric, or humanized antibody.

The term "conservative sequence modification" as used herein refers to an amino acid modification that does not significantly affect or alter the binding property of the antibody. Such conservative modifications include amino acid substitutions, additions and deletions. Modifications can be introduced into the antibodies of the present invention by standard techniques known in the art, such as point mutations and PCR-mediated mutations. Conservative amino acid substitutions are those in which an amino acid residue is replaced with an amino acid residue having a similar side chain. Groups of amino acid residues having similar side chains are known in the art. These groups of amino acid residues include amino acids with basic side chains (e.g., lysine, arginine, or histidine), amino acids with acidic side chains (e.g., aspartic acid or glutamic acid), amino acids with uncharged polar side chains (e.g., glycine, asparagine, glutamine, serine, threonine, tyrosine, cysteine, or tryptophan), amino acids with nonpolar side chains (e.g., alanine, valine, leucine, isoleucine, proline, phenylalanine, or methionine), amino acids with β-branched side chains (e.g., threonine, valine, or isoleucine), and amino acids with aromatic side chains (e.g., tyrosine, phenylalanine, tryptophan, or histidine). Therefore, one or more amino acid residues in CDR regions of the antibody of the present invention can be replaced with other amino acid residues in the group of amino acid residues with identical side chains, and the resulting antibody can be tested for retained functions (i.e., the functions described above) using the functional assays described herein.

### Genetically Modified Antibody

The antibody of the present invention may be prepared as a genetically modified antibody using an antibody having one or more V_{H}/V_{L} sequences of the Trop2 antibody of the present invention as a starting material. The antibody may be genetically modified by modifying one or more residues in one or two variable regions (i.e., V_{H} and/or V_{L}) (e.g., in one or more CDR regions and/or one or more framework regions) to improve binding affinity and/or increase similarity to naturally occurring antibodies of certain species. For example, the framework regions are modified to provide humanized antibodies. Furthermore, alternatively, the antibody may be genetically modified by modifying residues in the constant region, for example, to alter the effector function of the antibody. In certain embodiments, CDR grafting can be used to genetically modify variable regions of an antibody. The antibody mainly interacts with a target antigen through amino acid residues located in the six heavy and light chain complementarity determining regions (CDRs). For this reason, amino acid residues in CDRs are more diverse between individual antibodies than sequences outside the CDRs. Since the CDR sequences are responsible for the major antibody-antigen interactions, recombinant antibodies simulating the properties of a particular natural antibody can be expressed by constructing expression vectors containing CDR sequences of the particular natural antibody grafted into the framework sequences of different antibodies with different properties (Riechmann et al., (1998) Nature 332:323-327; Jones et al., (1986) Nature 321:522-525; Queen et al., (1989) Proc. Natl. Acad; U.S.A. 86:10029-10033; U.S. Pat. Nos. 5,225,539; 5,530,101; 5,585,089; 5,693,762; and 6,180,370).

Therefore, another embodiment of the present invention relates to an isolated monoclonal antibody or an antigen-binding moiety thereof, which comprises a heavy chain variable region comprising a CDR1, a CDR2, and a CDR3 having the above sequences of the present invention, and/or a light chain variable region comprising a CDR1, a CDR2, and a CDR3 having the above sequences of the present invention. Although these antibodies comprise CDR sequences of the V_{H} and V_{L} of the monoclonal antibody of the present invention, they may comprise different framework sequences.

Such framework sequences can be obtained from public DNA databases including germline antibody gene sequences or public references. For example, germline DNA sequences for human heavy and light chain variable region genes can be obtained in the Vbase human germline sequences database (www.mrc-cpe.cam.ac.uk/vbase) and Kabat et al., (1991), supra; Tomlinson et al., (1992) J. Mol. Biol. 227:776-798; and Cox et al., (1994) Eur. J. Immunol. 24:827-836. As another embodiment, germline DNA sequences for human heavy and light chain variable region genes can be found in the Genbank database. For example, the following heavy chain germline sequences in HCo7 HuMAb mice are under Genbank accession Nos. 1-69 (NG_0010109, NT_024637, and BC070333), 3-33 (NG_0010109 and NT_024637), and 3-7 (NG_0010109 and NT_024637). As another example, the following heavy chain germline sequences from Hco12 HuMAb mice are under Genbank accession Nos. 1-69 (NG_0010109, NT_024637, and BC070333), 5-51 (NG_0010109 and NT_024637), 4-34 (NG_0010109 and NT_024637), 3-30.3 (CAJ556644), and 3-23 (AJ406678).

Antibody protein sequences were compared to protein sequences in the database using one of the sequence similarity search methods known in the art as gapped BLAST (Altschul et al., (1997), supra).

Preferred framework sequences for the antibody of the present invention are those that are structurally similar to the framework sequences used in the antibody of the present invention. The CDR1, CDR2, and CDR3 sequences of the V_{H} may be grafted into framework regions having the same sequence as the germline immunoglobulin gene from which the framework sequences are derived, or the CDR sequences may be grafted into a framework region comprising one or more mutations compared to the germline sequence. For example, in some cases, it is beneficial to mutating residues in the framework region to retain or enhance the antigen-binding activity of the antibody (see, for example, U.S. Pat. Nos. 5,530,101; 5,585,089; 5,693,762; and 6,180,370).

Another class of variable region modifications is the mutation of amino acid residues in the CDR1, CDR2, and/or CDR3 regions of the V_{H} and/or V_{L} to improve one or more binding properties (e.g., affinity) of the target antibody. Mutations can be introduced by point mutations or PCR-mediated mutations, and the effect of the mutations on antibody binding or other functional properties can be evaluated through *in vitro* or *in vivo* assays known in the art. Preferably, conservative modifications known in the art are introduced. The mutation may be an amino acid substitution, addition, or deletion, and is preferably an amino acid substitution. Furthermore, generally no more than one, two, three, four, or five residues in the CDR regions are altered.

Furthermore, in another embodiment, the present invention provides an isolated Trop2 monoclonal antibody or an antigen-binding moiety thereof, which comprises a heavy chain variable region and a light chain variable region comprising: (a) a V_{H} CDR1 region comprising the sequence of the present invention, or an amino acid sequence with one, two, three, four, or five amino acid substitutions, deletions, or additions; (b) a V_{H} CDR2 region comprising the sequence of the present invention, or an amino acid sequence with one, two, three, four, or five amino acid substitutions, deletions, or additions; (c) a V_{H} CDR3 region comprising the sequence of the present invention, or an amino acid sequence with one, two, three, four, or five amino acid substitutions, deletions, or additions; (d) a V_{L} CDR1 region comprising the sequence of the present invention, or an amino acid sequence with one, two, three, four, or five amino acid substitutions, deletions, or additions; (e) a V_{L} CDR2 region comprising the sequence of the present invention, or an amino acid sequence with one, two, three, four, or five amino acid substitutions, deletions, or additions; and (f) a V_{L} CDR3 region comprising the sequence of the present invention, or an amino acid sequence with one, two, three, four, or five amino acid substitutions, deletions, or additions.

The genetically engineered antibodies of the present invention include those in which framework residues of the V_{H} and/or V_{L} have been genetically modified, for example, to alter antibody properties. Generally, these framework modifications are used to reduce the immunogenicity of the antibody. For example, one method is to "back mutate" one or more framework residues into the corresponding germline sequences. More specifically, an antibody undergoing a somatic mutation may contain framework residues that differ from the germline sequence of the resulting antibody. These residues can be recognized by comparing the framework sequences of the antibody to the germline sequences of the resulting antibody.

Another class of framework modifications includes the mutation of one or more residues in framework regions, or even one or more CDR regions, to remove T cell epitopes, thereby reducing the potential immunogenicity of the antibody. This method is also known as "deimmunization" and is described in more detail in U.S. Pat. Pub. No. 20030153043.

The present invention further includes active fragments, derivatives, and analogs of the anti-Trop2 antibody. As used herein, the terms "fragment", "derivative", and "analog" refer to a polypeptide that substantially retains the binding affinity function or activity of Trop2. The polypeptide fragments, derivatives, or analogs of the present invention may be (i) a polypeptide in which one or several conserved or non-conserved amino acid residues (preferably conserved amino acid residues) are substituted, or (ii) a polypeptide having substituents in one or more amino acid residues, or (iii) a polypeptide formed by fusing a CDR3-P1 polypeptide with another compound (such as a compound for prolonging the half-life of the polypeptide, e.g., polyethylene glycol), or (iv) a polypeptide formed by fusing an additional amino acid sequence with a sequence of the polypeptide (a fusion protein formed by fusion with a leader sequence, a secretory sequence, or a tag sequence such as 6His). These fragments, derivatives, and analogs are within the scope well known by those skilled in the art according to the teachings herein.

A preferred class of active derivatives refers to polypeptides formed by substitutions of up to 3, preferably up to 2, more preferably up to 1 amino acid with qualitatively similar or analogous amino acids as compared to the amino acid sequence of Table 1. These conservative variant polypeptides are preferably produced by performing amino acid substitutions according to Table 2.

**Table 2**

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gin |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| He (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

Furthermore, as an alternative to modifications in the framework or CDR regions, the antibody of the present invention may be genetically engineered to include genetic modifications in the Fc region, generally to alter one or more functional properties of the antibody, such as serum half-life, complement fixation, Fc receptor binding, and/or antibody-dependent cellular cytotoxicity. Furthermore, the antibody of the present invention may be chemically modified (e.g., one or more chemical functional groups may be attached to the antibody), or modified to alter the glycosylation of the antibody, or to alter one or more functional properties of the antibody.

In one embodiment, the hinge region of C_{H1} is modified to alter, e.g., to increase or decrease, the number of cysteine residues in the hinge region. This method is further described in U.S. Pat. No. 5,677,425. Cysteine residues in the C_{H1} hinge region are altered, for example, to facilitate assembly of the heavy chain light chain or to increase/decrease stability of the antibody.

In another embodiment, the Fc hinge region of the antibody is mutated to reduce the biological half-life of the antibody. More specifically, one or more amino acid mutations are introduced into the C_{H2}-C_{H3} linking region of the Fc hinge fragment such that the antibody has reduced SpA binding relative to natural Fc-hinge domain SpA binding. This method is described in more detail in U.S. Pat. No. 6,165,745.

In another embodiment, the glycosylation of the antibody is modified. For example, deglycosylated antibodies (i.e., antibodies lack glycosylation) can be prepared. Glycosylation can be altered, for example, to increase the affinity of the antibody for an antigen. Such glycosylation modifications can be achieved, for example, by altering one or more glycosylation sites in the sequence of the antibody. For example, one or more amino acid substitutions can be made to eliminate one or more glycosylation sites in framework regions of variable regions, thereby eliminating glycosylation at these sites. Such deglycosylation can increase the affinity of the antibody for an antigen. See, for example, U.S. Pat. Nos. 5,714,350 and 6,350,861.

Furthermore, antibodies with altered glycosylation patterns, e.g., low-fucosylated antibodies with reduced fucose residues or antibodies with increased bisecting GlcNac structures, can be prepared. The altered glycosylation patterns have been demonstrated to increase the ADCC activity of the antibodies. Such glycosylation modifications can be performed, for example, by expressing the antibodies in a host cell with an altered glycosylation system. Cells with altered glycosylation systems are known in the art and can be used as host cells for expressing the recombinant antibodies of the present invention to prepare antibodies with altered glycosylation. For example, cell lines Ms704, Ms705, and Ms709 lack the fucosyltransferase gene FUT8 (α(1,6)-fucosyltransferase), such that antibodies expressed in the Ms704, Ms705, and Ms709 cell lines lack fucose in their carbohydrates. The Ms704, Ms705, and Ms709 FUT8-/- cell lines are prepared by targeted disruption of the FUT8 gene using two alternative vectors in CHO/DG44 cells (see U.S. Pat. Pub. No. 20040110704 and Yamane-Ohnuki et al., (2004) Biotechnol Bioeng, 87:614-22). As another example, EP 1,176,195 documents a cell line in which the function of gene FUT8 is disrupted. The gene encodes a fucosyltransferase, such that an antibody expressed in the cell line exhibits low fucosylation by reducing or eliminating α-1,6 bond-related enzymes. EP 1,176,195 also describes a cell line with low or no enzymatic activity for adding fucose to N-acetylglucosamine binding to the Fc region of an antibody, e.g., the rat myeloma cell line YB2/0 (ATCC CRL 1662). WO 03/035835 describes a CHO variant cell line, Lec13 cell, which has reduced ability to add fucose to Asn (297)-related carbohydrates, resulting in low fucosylation of an antibody expressed in the host cell (see Shield et al., (2002) J. Biol. Chem. 277:26733-26740). Antibodies with altered glycosylation patterns can also be prepared in chicken eggs, as described in WO 06/089231. Alternatively, antibodies with altered glycosylation patterns can be prepared in plant cells such as duckweed. WO 99/54342 discloses a cell line genetically engineered to express a glycosyltransferase that modifies a glycoprotein (e.g., β(1,4)-*N*-acetylglucosaminyltransferase III (GnTIII)), such that an antibody expressed in the genetically engineered cell line exhibits an increased bisecting GlcNac structure that results in enhanced ADCC activity of the antibody (Umana et al., (1999) Nat. Biotech. 17:176-180). Alternatively, a fucosidase can be used to cut off a fucose residue of the antibody, e.g., an α-L-fucosidase removes a fucose residue from the antibody (Tarentino et al., (1975) Biochem. 14:5516-23).

Another modification of the antibodies herein is pegylation (PEGylation). The antibodies can be PEGylated, for example, to increase the biological (e.g., serum) half-life of the antibodies. To PEGylate an antibody, the antibody or the fragment thereof is generally reacted with polyethylene glycol (PEG), such as a reactive ester or aldehyde derivative of PEG, under conditions such that one or more PEG groups are attached to the antibody or antibody fragment. Preferably, PEGylation is performed by an acylation reaction or an alkylation reaction with a reactive PEG molecule (or similar reactive water-soluble polymer). The term "polyethylene glycol" as used herein includes any form of PEG used to derivatize other proteins, such as mono (C₁-C₁₀) alkoxy- or aryloxy polyethylene glycol or polyethylene glycol maleimide. In certain embodiments, the antibody to be PEGylated is a deglycosylated antibody. Methods for PEGylating proteins are known in the art and can be applied to the antibodies of the present invention. See, for example, EPO 154 316 and EP 0 401 384.

### Physical Properties of Antibody

The antibodies of the present invention can be characterized by their various physical properties, such that their classes are detected and/or distinguished.

For example, an antibody may comprise one or more glycosylation sites in the light or heavy chain variable region. These glycosylation sites may result in increased immunogenicity of the antibody, or altered pK values of the antibody due to altered antigen binding (Marshall et al., (1972) Annu Rev Biochem. 41:673-702; Gala and Morrison (2004) J Immunol. 172:5489-94; Wallick et al., (1988) J Exp Med. 168:1099-109; Spiro (2002) Glycobiology 12:43R-56R; Parekh et al., (1985) Nature 316:452-7; and Mimura et al., (2000) Mol Immunol. 37:697-706). Glycosylation is known to occur in motifs containing N-X-S/T sequences. In some cases, it is preferred that the Trop2 antibody does not contain variable region glycosylation. This can be achieved by selecting antibodies that do not contain glycosylation motifs in variable regions or by mutating residues in the glycosylation region.

In preferred embodiments, the antibody does not contain an asparagine isomerization site. Deamidation of asparagine may occur at the N-G or D-G sequence, creating isoaspartic acid residues which introduce knobs into the polypeptide chain and reduce its stability (isoaspartic acid effect).

Each antibody will have a unique isoelectric point (pI) substantially falling within the pH range of 6-9.5. The pI of IgG1 antibodies generally falls within a pH range of 7-9.5, while that of IgG4 antibodies substantially falls within a pH range of 6-8. It is speculated that antibodies with pI beyond the normal range may have some unfolding structures and be unstable under *in vivo* conditions. Therefore, it is preferred that the pI value of the Trop2 antibody falls within the normal range. This can be achieved by selecting antibodies with pI within the normal range or by mutating uncharged surface residues.

### Nucleic Acid Molecule Encoding the Antibody of the Present Invention

In another aspect, the present invention provides a nucleic acid molecule encoding heavy and/or light chain variable regions or CDRs of the antibody of the present invention. The nucleic acid may be present in an intact cell, in a cell lysate, or in a partially purified or substantially pure form. The nucleic acid is "isolated" or "substantially pure" when purified from other cellular components or other contaminants, such as other cellular nucleic acids or proteins, by standard techniques. The nucleic acid of the present invention may be, for example, DNA or RNA, and may or may not contain an intron sequence. In preferred embodiments, the nucleic acid is a cDNA molecule.

The nucleic acid of the present invention can be obtained using standard molecular biology techniques. For antibodies expressed by hybridomas (e.g., hybridomas prepared from transgenic mice carrying human immunoglobulin genes), cDNAs encoding the light and heavy chains of the antibodies prepared from the hybridomas can be obtained by standard PCR amplification or cDNA cloning techniques. For antibodies obtained from an immunoglobulin gene library (e.g., using phage display technique), nucleic acids encoding such antibodies can be collected from the gene library.

The preferred nucleic acid molecules of the present invention include those encoding the V_{H} and V_{L} sequences or CDRs of the Trop2 monoclonal antibody. Once the DNA fragments encoding the V_{H} and V_{L} are obtained, operations such as conversion of the variable region genes to full-length antibody chain genes, Fab fragment genes, or scFv genes can be further performed on these DNA fragments by standard recombinant DNA techniques. In these operations, the DNA fragment encoding the V_{H} or V_{L} is operably linked to another DNA fragment encoding another protein, such as an antibody constant region or a flexible linker. The term "operably linked" means that two DNA fragments are linked together such that the amino acid sequences encoded by the two DNA fragments are both in the reading frame.

Isolated DNA encoding the V_{H} region can be converted to a full-length heavy chain gene by operably linking V_{H}-encoding DNA to another DNA molecule encoding the heavy chain constant region (C_{H1}, C_{H2}, and C_{H3}). The sequence of the human heavy chain constant region gene is known in the art, and DNA fragments comprising these regions can be obtained by standard PCR amplification. The heavy chain constant region may be an IgG 1, IgG2, IgG3, IgG4, IgA, IgE, IgM, or IgD constant region, but is preferably an IgG1 or IgG4 constant region. For the Fab fragment heavy chain genes, DNA encoding the V_{H} region can be operably linked to another DNA molecule encoding only the heavy chain C_{H1} constant region.

Isolated DNA encoding the V_{L} region can be converted to a full-length light chain gene by operably linking V_{L}-encoding DNA to another DNA molecule encoding the light chain constant region C_{L}. The sequence of the human light chain constant region gene is known in the art, and DNA fragments comprising these regions can be obtained by standard PCR amplification. In preferred embodiments, the light chain constant region may be a κ or λ constant region.

To create the scFv gene, the DNA fragments encoding V_{H} and V_{L} may be operably linked to another fragment encoding a flexible linker, for example, encoding an amino acid sequence (Gly4-Ser)₃, such that the V_{H} and V_{L} sequences can be expressed as contiguous single-stranded proteins, wherein the V_{H} and V_{L} regions are linked through the flexible linker (see, for example, Bird et al., (1988) Science 242:423-426; Huston et al., (1988) Proc. Natl. Acad. Sci. USA 85:5879-5883; McCafferty et al., (1990) Nature 348:552-554).

### Preparation of the Monoclonal Antibody of the Present Invention

In the present invention, immunoglobulin antibodies comprising two heavy chains and two light chains with fully human variable regions were prepared using H2L2 transgenic mice from Harbour BioMed. Human heavy and light chain transgenic loci are introduced into mice using genetic engineering to produce human antibodies. Depending on the design of the introduced antibody heavy chain locus, the immune stimulation by a particular antigen can produce a class of conventional antibodies (e.g., IgG or IgA) with specific functional effects or multiple classes of antibodies (e.g., IgM and IgG).

Antigen-specific H2L2 monoclonal antibodies can be prepared using the somatic hybridization (hybridoma) technique in Kohler and Milstein (1975) Nature 256: 495. Other embodiments for preparing monoclonal antibodies include viral or oncogenic transformation of B lymphocytes and phage display techniques. Chimeric or humanized antibodies are also well known in the art. See, for example, U.S. Pat. Nos. 4,816,567; 5,225,539; 5,530,101; 5,585,089; 5,693,762; and 6,180,370.

### Production of Transfectoma for Preparing the Monoclonal Antibody of the Present Invention

The antibody of the present invention can also be produced in host cell transfectomas using, for example, a recombinant DNA technique in conjunction with a gene transfection method (e.g., Morrison, S. (1985) Science 229:1202). In one embodiment, DNA encoding partial or full-length light and heavy chains obtained by standard molecular biology techniques is inserted into one or more expression vectors such that the genes are operably linked to transcriptional and translational regulatory sequences. In this case, the term "operably linked" refers to the linkage of the antibody genes into the vector such that the transcriptional and translational control sequences within the vector perform their intended function of regulating the transcription and translation of the antibody genes.

The term "regulatory sequence" includes promoters, enhancers, and other expression control elements (e.g., polyadenylation signals) that control the transcription or translation of the antibody genes. Such regulatory sequences are described, for example, in Goeddel (Gene Expression Technology. Methods in Enzymology 185, Academic Press, San Diego, Calif. (1990)). Preferred regulatory sequences for expression in a mammalian host cell include viral elements that direct high-level protein expression in mammalian cells, e.g., promoters and/or enhancers derived from cytomegalovirus (CMV), simian virus 40 (SV40), adenovirus such as adenovirus major late promoter (AdMLP), and polyoma virus. Alternatively, non-viral regulatory sequences may be used, such as ubiquitin promoters or β-globin promoters. In addition, the regulatory elements are composed of sequences of different origins, for example, the SRα promoter system comprises the sequence from the SV40 early promoter and the long terminal repeat of the human T-cell leukemia type I virus (Takebe et al., (1988) Mol. Cell. Biol. 8:466-472). The expression vector and expression control sequences that are compatible with the expression host cell used are selected.

The antibody light chain gene and the antibody heavy chain gene can be inserted into the same expression vector or different expression vectors. In preferred embodiments, variable regions are inserted into an expression vector that has encoded the heavy chain constant region and the light chain constant region of the desired subtype to construct a full-length antibody gene, such that the V_{H} is operably linked to the C_{H} in the vector and the V_{L} is operably linked to the C_{L} in the vector. Alternatively, the recombinant expression vector can encode a signal peptide that facilitates the secretion of an antibody chain from a host cell. The antibody chain gene can be cloned into a vector such that the signal peptide is linked to the amino terminus of the antibody chain gene in the reading frame. The signal peptide may be an immunoglobulin signal peptide or a heterologous signal peptide (i.e., a signal peptide from a non-immunoglobulin protein).

In addition to the antibody chain genes and regulatory sequences, the recombinant expression vector of the present invention can carry other sequences, such as a sequence that regulates replication of the vector in the host cell (e.g., an origin of replication) and a selectable marker gene. The selectable marker gene can be used to select a host cell into which the vector has been introduced (see, e.g., U.S. Pat. Nos. 4,399,216; 4,634,665; and 5,179,017). For example, the selectable marker gene generally confers drug resistance, e.g., G418, hygromycin, or methotrexate resistance, on the host cell into which the vector has been introduced. Preferred selectable marker genes include a dihydrofolate reductase (DHFR) gene (for methotrexate selection/amplification in dhfr host cells) and a neo gene (for G418 selection).

For expression of the light and heavy chains, the expression vectors encoding the heavy and light chains are transfected into the host cell by standard techniques. The term "transfection" in its various forms encompasses a variety of techniques commonly used to introduce exogenous DNA into prokaryotic or eukaryotic host cells, e.g., electroporation, calcium phosphate precipitation, DEAE-dextrose transfection, and the like. Although expressing the antibody of the present invention in prokaryotic or eukaryotic host cells is theoretically feasible, expressing the antibody in eukaryotic cells is preferred, and expressing the antibody in mammalian host cells is the most preferred. This is because that eukaryotic cells, and in particular mammalian cells, are more likely than prokaryotic cells to assemble and secrete a properly folded and immunologically active antibody.

Preferred mammalian host cells for expressing the recombinant antibody of the present invention include Chinese hamster ovary cells (CHO cells) (including dhfr-CHO cells administered with a DHFR selectable marker, which are described in Urlaub and Chasin, (1980) Proc. Natl. Acad. Sci. USA 77:4216-4220; the DHFR selectable marker is described, for example, in R. J. Kaufman and P. A. Sharp (1982) J. Mol. Biol. 159:601-621), NSO myeloma cells, COS cells, and SP2 cells. Particularly when using NSO myeloma cells, another preferred expression system is a GS gene expression system, which is described in WO 87/04462, WO 89/01036, and EP 338,841. When a recombinant expression vector encoding an antibody gene is introduced into a mammalian host cell, the antibody is prepared by culturing the host cell for a period of time sufficient to allow expression of the antibody in the host cell, or preferably sufficient to allow secretion of the antibody into a medium in which the host cell is grown. The antibody can be isolated from the medium using protein purification methods.

### Recombinant Protein and Preparation Method Therefor

The present invention further relates to a recombinant protein comprising the antibody or the antigen-binding protein of the present invention, or a recombinant protein comprising an amino acid sequence having at least 50%, preferably at least 70%, and more preferably at least 80% sequence identity to the antibody or the antigen-binding protein of the present invention. Furthermore, the recombinant protein of the present invention may further include a fusion protein formed by fusing the nanobody of the present invention with an expression tag (e.g., 6His).

The relevant sequence, once obtained, can be replicated in large amount by recombination. This is generally implemented by cloning the sequence into a vector, transferring the vector into a cell, and then isolating the relevant sequence from proliferated host cells by conventional methods. Biomolecules (nucleic acids, proteins, etc.) to which the present invention relates include those present in an isolated form.

At present, the DNA sequence encoding the recombinant protein of the present invention (or the fragment thereof, or the derivative thereof) can be obtained completely by chemical synthesis. The DNA sequence can then be introduced into various existing DNA molecules (or such as vectors) and cells known in the art. Furthermore, mutations can also be introduced into the sequence of the recombinant protein of the present invention by chemical synthesis.

The present invention further relates to a vector comprising the suitable DNA sequence described above and an appropriate promoter or control sequence. These vectors can be used to transform appropriate host cells, to enable them to express proteins.

The host cells may be prokaryotic cells, such as bacterial cells; or lower eukaryotic cells, such as yeast cells; or higher eukaryotic cells, such as mammalian cells. Representative examples include bacterial cells of *Escherichia coli, Streptomyces* spp. and *Salmonella typhimurium;* fungal cells such as yeast; insect cells such as *Drosophila* S2 or Sf9 cells; animal cells such as CHO, COS7, 293 cells.

Transformation of host cells with recombinant DNA may be performed by conventional techniques well known to those skilled in the art. When the host is a prokaryote, such as E. *coli,* competent cells capable of absorbing DNA can be harvested after exponential phase and processed with CaCl₂ according to steps that are well known in the art. Another method is to use MgCl₂. If necessary, the transformation can also be performed by electroporation. When the host is a eukaryote, the following DNA transfection methods can be used: calcium phosphate coprecipitation, conventional mechanical methods such as microinjection, electroporation, liposome packaging, and the like.

The obtained transformant can be cultured by conventional methods to express the polypeptide encoded by the gene of the present invention. The medium used in the culturing may be selected from various conventional media depending on the host cell used. The culturing is performed under conditions suitable for the growth of the host cells. After the host cells have been grown to an appropriate cell density, the selected promoter is induced by suitable methods (e.g., temperature conversion or chemical induction) and the cells are cultured for an additional period of time.

The recombinant polypeptide in the above method may be expressed intracellularly, or on the cell membrane, or secreted extracellularly. If necessary, the recombinant protein can be separated and purified by various isolation methods according to physical, chemical, and other properties. These methods are well known to those skilled in the art. Examples of these methods include, but are not limited to: conventional renaturation treatment, treatment with a protein precipitant (such as salt precipitation), centrifugation, osmotic lysis, sonication treatment, ultracentrifugation, molecular sieve chromatography (gel filtration), adsorption chromatography, ion exchange chromatography, high performance liquid chromatography (HPLC), and other various liquid chromatography techniques, and combinations thereof.

### Immunoconjugate

The antibody of the present invention can be cross-linked to a therapeutic agent to form an immunoconjugate, such as an antibody-drug conjugate (ADC). Suitable therapeutic agents include cytotoxins, alkylating agents, DNA minor groove binding molecules, DNA intercalators, DNA cross-linking agents, histone deacetylase inhibitors, nuclear export inhibitors, proteasome inhibitors, inhibitors of topoisomerase I or II, heat shock protein inhibitors, tyrosine kinase inhibitors, antibiotics and antimitotic agents, preferably SN-38. In ADCs, the antibody and therapeutic agent are preferably cross-linked by a linker that is cleavable, e.g., a peptide linker, a disulfide linker, or a hydrazone linker. More preferably, the linker is a peptide linker, such as Val-Cit, Ala-Val, Val-Ala-Val, Lys-Lys, Pro-Val-Gly-Val-Val, Ala-Asn-Val, Val-Leu-Lys, Ala-Ala-Asn, Cit-Cit, Val-Lys, Lys, Cit, Ser, or Glu. ADCs can be prepared as described in U.S. Pat. Nos. 7,087,600; 6,989,452; and 7,129,261; PCT publication Nos. WO 02/096910; WO 07/038,658; WO 07/051,081; WO 07/059,404; WO 08/083,312; and WO 08/103,693; U.S. Pat. Nos. 20060024317, 20060004081, and 20060247295.

### Multispecific Antibody

In another aspect, the present invention relates to a multispecific antibody comprising one or more antibodies of the present invention linked to at least one other functional molecule, such as another peptide or protein (e.g., another antibody or receptor ligand), to produce a multispecific antibody that binds to at least two different binding sites or targeted molecules. The term "multispecific antibody" includes antibodies having three or more specificities.

In embodiments, the bispecific molecule has a third specificity in addition to the Fc binding specificity and the Trop2 binding specificity. The third specificity may be for an enhancement factor (EF), e.g., a molecule that binds to a surface protein involved in cytotoxic activity and thereby increases an immune response against the target cell. For example, an enhancement factor antibody can bind to a cytotoxic T cell (e.g., through CD2, CD3, CD8, CD28, CD4, CD40, or ICAM-1) or other immune cells, resulting in an enhanced immune response against the target cell.

The multispecific antibody may be present in a variety of forms and sizes. At one end of the size spectrum, the multispecific antibody remains in the conventional antibody format, except that it has two binding arms with different types of specificity instead of having two binding arms with the same specificity. At the other extreme are bispecific molecules consisting of two single-chain antibody fragments (scFvs) linked by a peptide chain, called Bs(scFv)₂ constructs. Medium-sized bispecific molecules comprise two different F(ab) fragments linked by a peptide linker. These and other forms of bispecific molecules can be prepared by genetic engineering, somatic hybridization, or chemical methods. See, for example, Kufer et al., cited supra; Cao and Suresh, Bioconjugate Chemistry, 9 (6), 635-644 (1998); and van Spriel et al., Immunology Today, 21 (8), 391-397 (2000).

### Oncolytic Virus Encoding or Carrying Antibody

Oncolytic viruses preferentially infect and kill cancer cells. The antibody of the present invention is used with oncolytic viruses. Furthermore, an oncolytic virus encoding the antibody of the present invention can be introduced into a human.

### Pharmaceutical Composition

In another aspect, the present invention provides a pharmaceutical composition comprising one or more antibodies of the present invention formulated together with a pharmaceutically acceptable carrier. The composition may optionally comprise one or more other pharmaceutically active ingredients, such as another antibody, a chemotherapeutic agent, and the like. The pharmaceutical compositions of the present invention may be administered in a combination therapy with, for example, another anti-cancer agent, another anti-inflammatory agent, or a vaccine.

The pharmaceutical composition may comprise any number of excipients. Excipients that may be used include carriers, surfactants, thickening or emulsifying agents, solid binders, dispersing or suspending agents, solubilizers, colorants, flavoring agents, coatings, disintegrants, lubricants, sweeteners, preservatives, isotonic agents, and combinations thereof. Selection and use of suitable excipients is taught in Gennaro, ed., Remington: The Science and Practice of Pharmacy, 20th Ed. (Lippincott Williams & Wilkins 2003).

Preferably, the pharmaceutical composition is suitable for intravenous, intramuscular, subcutaneous, parenteral, spinal, or epidermal administration (e.g. by injection or bolus injection). Depending on the route of administration, the active ingredient may be encapsulated in a material to protect it from acids and other natural conditions that may inactivate it. "Parenteral administration" refers to a mode that is different from enteral administration and topical administration and that is generally performed by injection, including but not limited to, intravenous, intramuscular, intra-arterial, intramembranous, intracapsular, intraorbital, intracardiac, intradermal, intraperitoneal, transtracheal, subcutaneous, subcuticular, intra-articular, subcapsular, subarachnoid, intraspinal, epidural and intrasternal injection and bolus injection. Alternatively, the antibody of the present invention may be administered by a non-parenteral route, such as topical, epidermal, or mucosal administration, such as intranasal, oral, vaginal, rectal, sublingual, or topical administration.

The pharmaceutical compositions may be in the form of sterile aqueous solutions or dispersions. They may also be formulated in microemulsions, liposomes or other ordered structures suitable for high concentrations of drugs.

The amount of active ingredient that is formulated together with a carrier material into a single dosage form will vary with a subject treated and a particular mode of administration, and is essentially the amount of the composition that produces a therapeutic effect. The amount of the active ingredient in combination with a pharmaceutically acceptable carrier is, by percentage, about 0.01% to about 99%, preferably about 0.1% to about 70%, and most preferably about 1% to about 30%.

The administration regimen is adjusted to provide the optimal desired response (e.g., therapeutic response). For example, a rapid perfusion agent may be administered, multiple divided doses may be administered over time, or the dose may be reduced or increased in proportion to the criticality of the treatment situation. It is particularly advantageous to formulate parenteral compositions in dosage units for ease of administration and uniformity of dosage. Dosage unit form refers to physically separate units suitable for single administration to a subject treated, each unit containing a predetermined amount of active ingredient calculated to produce the desired therapeutic effect together with the pharmaceutical carrier. Alternatively, the antibody may be administered in a sustained-release formulation, in which case the frequency of administration required is reduced.

For administration of the antibody, the dosage may be about 0.001-100 mg/kg host body weight, more usually 0.01-5 mg/kg host body weight. For example, the dose may be 0.3 mg/kg body weight, 1 mg/kg body weight, 3 mg/kg body weight, 5 mg/kg body weight, or 10 mg/kg body weight, or in the range of 1-10 mg/kg body weight. Exemplary treatment regimens involve administration once a week, once every two weeks, once every three weeks, once every four weeks, once a month, once every 3 months, or once every 3-6 months. Preferred administration regimens for Trop2 of the present invention include intravenous administration, 1 mg/kg body weight or 3 mg/kg body weight, with the antibody being administered on one of the following dosing schedules: (i) six times every four weeks and then once every three months; (ii) once every three weeks; (iii) once at 3 mg/kg body weight and then once every three weeks at 1 mg/kg body weight. In some methods, the dosage is adjusted to achieve a plasma concentration of about 1-1000 µg/mL, in some methods, about 25-300 µg/mL.

A "therapeutically effective amount" of the Trop2 antibody of the present invention causes a reduction in the severity of symptoms of the disease, an increase in the frequency and duration of asymptomatic phases, or the ability to prevent damage or disability caused by susceptibility to the disease. For example, for treatment of a subject with a tumor, a "therapeutically effective amount" preferably inhibits tumor growth by at least about 20%, more preferably by at least about 40%, even more preferably by at least about 60%, and more preferably by at least about 80%, relative to an untreated subject. A therapeutically effective amount of a therapeutic antibody can reduce tumor size, or alleviate a symptom in a subject, which may be a human or another mammal.

The pharmaceutical composition may be a sustained-release agent, including implants, transdermal patches, and microencapsulated delivery systems. Biodegradable and biocompatible polymers such as ethylene-vinyl acetate, polyanhydrides, polyglycolic acid, collagen, polyorthoesters and polylactic acid may be used. See, for example, Sustained and Controlled Release Drug Delivery Systems, J. R. Robinson, ed., Marcel Dekker, Inc., New York, 1978.

The pharmaceutical compositions can be administered by a medical device, such as (1) a needleless hypodermic injection device (e.g., U.S. Pat. Nos. 5,399,163; 5,383,851; 5,312,335; 5,064,413; 4,941,880; 4,790,824; and 4,596,556); (2) a micro-infusion pump (U.S. Pat. No. 4,487,603); (3) a transdermal administration device (U.S. Pat. No. 4,486,194); (4) a bolus injection device (U.S. Pat. Nos. 4,447,233 and 4,447,224); and (5) an osmotic device (U.S. Pat. Nos. 4,439,196 and 4,475,196).

In certain embodiments, the monoclonal antibody of the present invention can be formulated to ensure appropriate *in vivo* distribution. For example, to ensure that the therapeutic antibody of the present invention crosses the blood-brain barrier, the antibody may be formulated in liposomes, which may additionally contain targeting functional groups to enhance selective delivery to particular cells or organs. See, for example, U.S. Pat. Nos. 4,522,811; 5,374,548; 5,416,016; and 5,399,331; V. V. Ranade (1989) J. Clin.Pharmacol.29:685*;* Umezawa et al., (1988) Biochem. Biophys. Res. Commun. 153:1038; Bloeman et al., (1995) FEBS Lett.357:140*;* M. Owais et al., (1995) Antimicrob. Agents Chemother. 39:180; Briscoe et al., (1995) Am. J. Physiol. 1233:134; Schreier et al., (1994) J. Biol. Chem. 269:9090; Keinanen and Laukkanen (1994) FEBS Lett. 346:123; and Killion and Fidler (1994) Immunomethods 4:273.

### Use and Method of the Present Invention

The antibody (the composition, bispecific molecule, and immunoconjugate) of the present invention has a variety of *in vitro* and *in vitro* applications involving, for example, the diagnosis and/or treatment of cancer. The antibody can be administered to a human subject to, for example, inhibit tumor growth *in vivo.* In the diagnosis of cancer, a target tissue sample may be collected and contacted with the antibody of the present invention, wherein if a high level of Trop2 is detected in certain regions or cell classes, the subject is diagnosed as possibly having cancer, and an increase/decrease in the expression of Trop2 indicates cancer progression/remission.

In view of the ability of the Trop2 antibody of the present invention to inhibit the proliferation and survival of tumor cells, the present invention provides a method for inhibiting the growth of tumor cells in a subject, which comprises administering to the subject the antibody of the present invention, whereby tumor growth is inhibited in the subject. Non-limiting examples of tumors that can be treated by the antibody of the present invention include, but are not limited to, breast cancer, gastric cancer, pancreatic cancer, ovarian cancer, and intestinal cancer, either primary or metastatic. Furthermore, refractory or recurrent malignant tumors may be inhibited with the antibody of the present invention. These and other methods of the present invention are discussed further below.

### Combination Therapy

The present invention provides a combination therapy of the Trop2 antibody or the antigen-binding moiety thereof of the present invention administered with one or more additional antibodies, which is effective to inhibit tumor growth in a subject. In one embodiment, the present invention provides a method for inhibiting tumor growth in a subject, which comprises administering to the subject the Trop2 antibody and one or more additional antibodies, e.g., a LAG-3 antibody, a PD-1 antibody, and/or a CTLA-4 antibody. In certain embodiments, the subject is a human. In another aspect, the present invention provides a method for treating cancer, wherein the Trop2 antibody or the antigen-binding moiety thereof of the present invention is administered with a chemotherapeutic agent, which may be a cytotoxic agent. For example, SN-38, epirubicin, oxaliplatin, and/or 5-FU can be administered to a patient receiving a Trop2 antibody therapy.

Other therapies that may be combined with the Trop2 antibody include, but are not limited to, administration of an immunogenic agent, administration of interleukin 2 (IL-2), radiotherapy, surgery, or hormone deprivation.

The combination of therapeutic agents discussed herein can be administered simultaneously as a single composition in a pharmaceutically acceptable carrier, or as separate compositions, wherein each agent is in a pharmaceutically acceptable carrier. In another embodiment, the combination of therapeutic agents can be administered sequentially.

Furthermore, if multiple administrations of the combination therapy are performed and the agents are administered sequentially, the order of sequential administration at each time point may be reversed or maintained, and the sequential administration may be combined with simultaneous administration or any combination thereof.

### Kit

The present invention further provides a kit comprising the antibody (or the fragment thereof) or the assay plate of the present invention, and in one preferred embodiment of the present invention, the kit further comprises a container, instructions for use, a buffer, and the like.

The present invention further provides an assay kit for detecting the level of Trop2, which comprises an antibody for identifying the Trop2 protein, a lysis medium for dissolving a sample, and universal reagents and buffers required by detection, such as various buffers, detection markers, and detection substrates. The assay kit may be an *in vitro* diagnostic device.

### The main advantages of the present invention include that:

The present application provides a fully human antibody against a Trop2-binding protein, which has at least one of the following properties:
1) capable of specifically binding to HEK293 cells overexpressing human Trop2, CHO-K1 cells overexpressing monkey Trop2, or a Trop2 protein on the surface of BxPC-3 cells in a FACS assay;
2) capable of internalizing and killing BxPC-3 cells after conjugation with MMAF;
3) having ADCP activity against BxPC-3 cells;
4) having ADCC activity against BxPC-3 cells;
5) having a binding epitope different from that of the reference antibody hRS7; and
6) capable of being endocytosed by tumor cells BxPC-3 or HEK293 cells over-expressing human Trop2 in a short time.

The present invention will be further illustrated with reference to the following specific examples. It should be understood that these examples are merely intended to illustrate the present invention rather than limit the scope of the present invention. Experimental procedures without specific conditions indicated in the following examples are generally performed following conventional conditions, such as conditions described in Sambrook et al., Molecular Cloning: Laboratory Manual (New York: Cold Spring Harbor Laboratory Press, 1989), or conditions recommended by the manufacturers. Unless otherwise indicated, percentages and parts are by weight.

### Example 1: H2L2 Mouse Immunization, Hybridoma Cell Fusion, and Antibody Screening

H2L2 transgenic mice (WO2010/070263 A1) can generate an immune response and antibody titer comparable to wild-type mice (such as BALB/C). Antibodies against human Trop2 are produced by immunization of H2L2 mice with two immunogens (human Trop2 ECD-hFc protein and HEK293T/hTrop2 cell strain).

### 1.1. Trop2 ECD-hFc protein immunization

Six- to eight-week-old Harbour H2L2 transgenic mice were immunized with a recombinant glycosylated human Trop2 ECD-hFc protein (Chempartner, Shanghai) as an immunogen, and bred under specific pathogen free (SPF) conditions. In the first immunization, 50 µg of immunogenic protein and 0.2 mL of complete Freund's adjuvant (CFA, Sigma, #F5881) were injected into the abdominal cavity and axillary and inguinal lymph nodes of each mouse. To enhance the immune response, two weeks after the first immunization, 25 µg of immunogenic protein and 200 µL of Ribi (Sigma adjuvant system, Sigma, #S6322) were injected into the abdominal cavity and subcutaneous lymph nodes of each mouse, followed by the injection of 25 µg of the immunogen and 200 µL of Ribi adjuvant every 2 weeks, for a total of 6 times including the first immunization. One week after each of the fourth and sixth injections during the immunization, mouse blood was collected and subjected to a 10-fold dilution to obtain 5 concentrations (1:100, 1:1000, 1:10,000, 1:100,000, and 1:1,000,000). The titer of human Trop2 in mouse blood was determined by an ELISA assay in an ELISA plate coated with a human Trop2 Fc protein (Chempartner, Shanghai), and the specific reactivity of mouse blood at 2 concentrations (1:100 and 1:1000) for CHO-K1/hTrop2 cells (Chempartner, Shanghai) highly expressing Trop2 and CHO-K1 blast cells was assayed by flow cytometry. Serum of mice before immunization was used as a blank control group (PB).

### 1.2. Immunization with stably transfected cell strain HEK293/hTrop2 expressing Trop2

Six- to eight-week-old Harbour H2L2 transgenic mice were immunized with a stably transfected cell strain highly expressing human Trop2, HEK293/hTrop2 (Chempartner, Shanghai), as an immunogen, and bred in specific pathogen free (SPF) conditions. In the first immunization, 2×10⁶ HEK293/Trop2 cells as an immunogen and 0.2 mL of complete Freund's adjuvant (CFA, Sigma, #F5881) were injected into the abdominal cavity, axilla, and inguen. To enhance the immune response, two weeks after the first immunization, 2×10⁶ HEK293/Trop2 cells as an immunogen and 200 µL of Ribi (Sigma adjuvant system, Sigma, #S6322) were injected into the abdominal cavity and subcutaneous lymph nodes of each mouse, followed by the injection of 2×10⁶ cells as the immunogen and 200 µL of Ribi adjuvant every 2 weeks, for a total of 6 times including the first immunization. One week after each of the fourth and sixth injections during the immunization, mouse blood was collected and subjected to a 10-fold dilution to obtain 5 concentrations (1:100, 1:1000, 1:10,000, 1:100,000, and 1:1,000,000). The titer of human Trop2 in mouse blood was determined by an ELISA assay in an ELISA plate coated with a human Trop2 Fc protein (Chempartner, Shanghai), and the specific reactivity of mouse blood at 2 concentrations (1:100 and 1:1000) for CHO-K1/hTrop2 cells (Chempartner, Shanghai) highly expressing Trop2 and CHO-K1 blast cells was assayed by flow cytometry. Serum of mice before immunization was used as a blank control group (PB).

After completion of the above steps, mice with a specific immune response to human Trop2 were selected for a booster immunization by intraperineal injection of 100 µg of purified human Trop2 Fc (Chempartner, Shanghai, #151215003) protein prior to fusion. Three days later, the mice were sacrificed, from which spleen cells and lymph node cells were collected. NH₄OH was added to the spleen cell and lymph node samples to make a final concentration of 1% (w/w) to lyse red blood cells in the samples. The samples were centrifuged at 1000 rpm and washed three times with a DMEM medium, and the survival rate and number of cells were determined. Mouse myeloma cells sp2/0 (ATCC, #CRL-1581) were washed twice with serum-free DMEM, and the survival rate and number of cells were determined. Living spleen cells were then fused with mouse myeloma cells sp2/0 (ATCC, #CRL-1581) at a ratio of 4:1 by an efficient electrofusion method.

The fused cells were resuspended in a medium (Hybridoma-SFM, #12045084, Life Technologies) containing 20% ultra-low IgG FBS (ultra-low IgG, Fetal Bovine Serum, #16250086, Life Technologies) supplemented with 1× hypoxanthine, aminopterin, and thymidine (50× HAT supplement, #21060017, Life Technologies) and adjusted to a concentration of 10⁵ cells/200 µL. 200 µL of fusion cells were added to each well of a 96-well plate and cultured at 37 °C with 5% CO₂. 14 days after cell fusion, the hybridoma supernatant obtained with a glycosylated Trop2 Fc protein as an immunogen was screened by determining its ability to bind to a human Trop2 Fc protein by an enzyme-linked immunosorbent assay (ELISA); and the hybridoma supernatant obtained with HEK293/hTrop2 as an immunogen was assayed for its ability to bind to CHO-K1/hTrop2 and CHO-K1 blast cells in an Acumen fluorescence microplate.

The positive clones selected (OD450 > 2) or those with a positive percentage of > 80% in Acumen were screened by flow cytometry for clones which specifically bind to CHO-K1/human Trop2 cells, CHO-Kl/monkey Trop2 cells, and CHO-K1/mouse Trop2 cells (Chempartner,Shanghai). After fluorescence intensity scoring, 70 hybridoma parent clones with the strongest fluorescence intensity were selected and subcloned by a limiting dilution method. The submonoclonals grown after screening were screened by ELISA, Acumen, and flow cytometry to find the strongest submonoclonals binding to human, monkey, and mouse Trop2 cells. Submonoclonals, determined as an IgG subtype by mouse Ig typing Ready-SET-GO! ELISA (Life technologies, #88-50640-88), were sequenced for analysis.

### Example 2: Sequencing, Expression, and Purification of Monoclonal Trop2 Antibodies

The monoclonal Trop2 antibodies were sequenced, with the sequences summarized in Table 1a and Table 1b.

The heavy chain variable region sequence of the Trop2 antibody was subcloned into a pTT5 expression vector containing a signal peptide and the human heavy chain IgG1 constant region. The light chain variable region sequence of the Trop2 antibody was subcloned into an expression vector containing a signal peptide and the human antibody light chain kappa constant region. The recombinant plasmids were confirmed by sequencing and extracted with a Maxi kit (Macherey-Nagel, NucleoBond^{®} Xtra Midi) to improve the purity and quality of the recombinant plasmids, which were then filtered through a 0.22 µm filter (millpore). The purified plasmids were used for transfection. 293-6E cells (Genescript, Nanjing, China) were cultured in a FreeStyle 293 medium (Invitrogen, #12338026) in an incubator at 37 °C and 130 rpm with 5% CO₂. Transfection was performed after the HEK293-6E cells were adjusted to a cell density of 1-1.5×10⁶ cells/mL. The HEK293-6E cells were co-transfected with heavy and light chain plasmids by PEI (sigma) for one week. The titer of the antibody was determined approximately on days 5-7. The HEK293E culture was centrifuged (30 min, 3500 rpm) approximately on days 6-7, and the supernatant was collected and purified by filtration through a 0.22 µm filter.

A protein A column (GE) was washed with 0.1 M NaOH for 30 min or 5 column volumes of 0.5 M NaOH to remove endotoxin. A protein A column that has not been used for a long time was firstly soaked in 1 M NaOH for at least 1 h, then washed with endotoxin-free water until the pH value was neutral, and finally washed with 10 column volumes of 1% Triton X-100. The protein column was then equilibrated with 5 column volumes of phosphate buffered saline PBS (pH 7.4). The supernatant collected above was loaded on the column, and the flow-through liquid was collected as necessary. The column was washed with 5 column volumes of PBS, and then 5 column volumes of 0.1 M glycine-HCl (pH 3.0) was added for elution. The eluate containing the Trop2 antibody was neutralized with 0.5 column volumes of 1 M Tris-HCl (NaCl 1.5 M), pH 8.5. The human anti-Trop2 antibody was dialyzed in 1× PBS for 4 h to prevent endotoxin contamination. After dialysis, the concentration of the anti-Trop2 antibody was determined by spectrophotometry or a kit, the purity of the antibody was determined by high performance liquid chromatography-mass spectrometry, and the content of endotoxin was determined by a portable endotoxin test instrument (Endosafe^{®} nexgen-PTS^{™}, Charles River).

Through the above experiment, a total of 29 Trop2 monoclonal antibodies with unique sequences were obtained. The variable region sequences of the antibodies are listed in Table 1a. The obtained 29 antibodies were all of the IgG1 subtype, with the germline of both chains differing from that of the reference antibody Tab (hRS7).

Furthermore, two comparative example antibodies were prepared for use in the subsequent examples, one being a comparative example 1 antibody PR001131 (in-house) and the other being a comparative example 2 antibody Tab (hRS7, ChemPartner, #T07-001).

Specifically, for the constant region sequences and the variable region sequences of the comparative example 1 antibody PR00 1131, the amino acid sequence of VH is set forth in SEQ ID NO: 193, the amino acid sequence of VL is set forth in SEQ ID NO: 223, the amino acid sequence of heavy chain is set forth in SEQ ID NO: 242, and the amino acid sequence of light chain is set forth in SEQ ID NO: 272.

The comparative example 2 antibody Tab (hRS7) is a comparative example antibody prepared on the basis of sacituzumab govitecan from Immunomedics, Inc. and Seattle Genetics, and the amino acid sequences of the heavy and light chains are set forth in SEQ ID NO: 290 and SEQ ID NO: 291, respectively.

### Example 3: Binding Ability of Recombinant Trop2 Antibodies to 293T-Human Trop2

293T-human Trop2 cells (KyInno, #KC-0995) were digested with 3 mL of TrypLE enzyme (Life technologies, #12604-013), and then the digestion was stopped with 7 mL of corresponding medium. The cell density was determined, and the cells were resuspended to 1×10⁶ cells/mL in PBS. 100 µL of cell suspension was taken from each well and added to a 96 well V-bottom plate. The 96-well V-bottom plate was centrifuged at 1000 rpm for 5 min and washed once with PBS + 0.5% BSA (VWR, #0332-100G). The cells were resuspended in 100 µL of antibody diluted in a gradient (5-fold dilution, starting concentration: 66.67 nM) and incubated at 4 °C for 1 h, with hRS7 (ChemPartner, #T07-001) and hIgG 1 as reference and negative controls, respectively. After 1 h, the cells were washed twice with PBS + 0.5% BSA. The cells were incubated with 100 µL of Alexa Fluor 488 goat anti-human IgG (Life technologies, 1:1000, #A-11013) at 4 °C for 1 h in the dark, and washed twice with PBS + 0.5% BSA. The cells were resuspended in 200 µL of PBS + 0.5% BSA. The fluorescence intensity of the cells was analyzed using a FACS verse flow cytometer. All procedures were performed on ice.

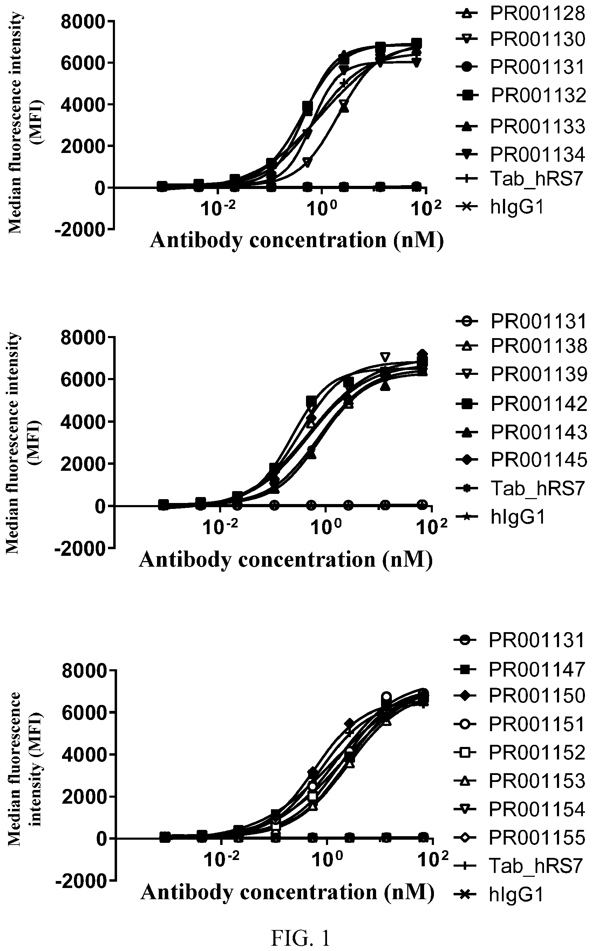

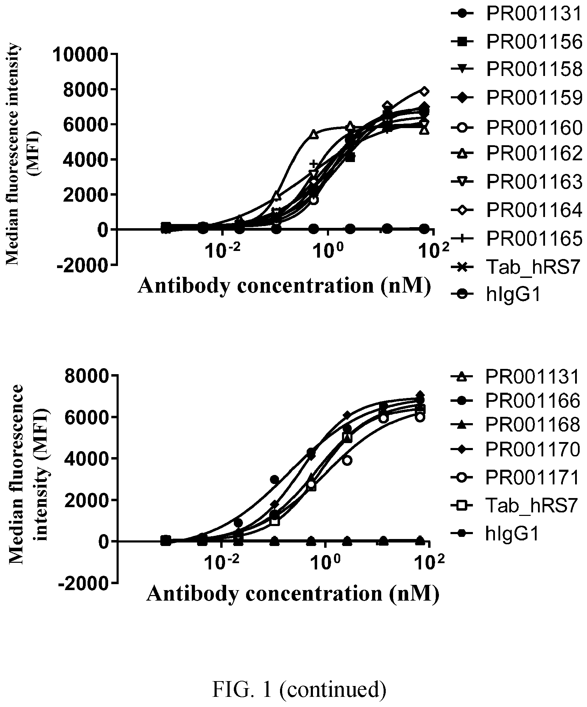

As shown in FIG. 1 and Table 3, the 29 Trop2 antibodies of the present invention all specifically bound to human Trop2, and the detected binding ability of the antibodies increased in a positive correlation with the antibody concentration. In contrast, the comparative example 1 antibody PR001131 had very weak binding to human Trop2. Compared to the comparative example 2 antibody Tab (hRS7), at the same concentration, the 26 Trop2 antibodies of the present invention exhibited MFI max higher than or comparable to the reference antibody hRS7, indicating that the antibodies were capable of binding to more hTrop2 proteins on HEK293/hTrop2 cells. Among them, 14 antibodies had EC₅₀ lower than the reference antibody, indicating that these antibodies were capable of binding to Trop2 more sensitively at a lower concentration, and among them, PR001142, PR001162, and PR001166 were the best and all had EC₅₀ less than 0.3 nM, about three times less than the EC₅₀ of the reference antibody.

**Table 3. Binding ability of Trop2 antibodies to 293T-human Tron2**

| Antibody | EC₅₀(nM) | Maximum (MFI) | Top% |
|---|---|---|---|
| PR001128 | 0.466 | 6846 | 105.66 |
| PR001130 | 2.057 | 6819 | 105.25 |
| PR001131 | 22.62 | 68.35 | 1.05 |
| PR001132 | 0.441 | 6923 | 106.85 |
| PR001133 | 0.995 | 7198 | 111.1 |
| PR001134 | 0.653 | 6035 | 93.15 |
| PR001138 | 0.478 | 6726 | 103.81 |
| PR001139 | 0.325 | 6846 | 105.66 |
| PR001142 | 0.23 | 6512 | 100.51 |
| PR001143 | 0.795 | 6299 | 97.22 |
| PR001145 | 0.48 | 7026 | 108.44 |
| PR001147 | 1.314 | 7422 | 114.55 |
| PR001150 | 0.589 | 6571 | 101.42 |
| PR001151 | 1.608 | 7511 | 115.93 |
| PR001152 | 1.929 | 7185 | 110.9 |
| PR001153 | 2.466 | 6937 | 107.07 |
| PR001154 | 2.106 | 7470 | 115.3 |
| PR001155 | 2.541 | 7319 | 112.96 |
| PR001156 | 1.667 | 7271 | 112.22 |
| PR001158 | 1.257 | 6952 | 107.3 |
| PR001159 | 0.977 | 6788 | 104.77 |
| PR001160 | 1.224 | 6713 | 103.61 |
| PR001162 | 0.158 | 5847 | 90.25 |
| PR001163 | 0.496 | 5988 | 92.42 |
| PR001164 | 2.396 | 8949 | 138.12 |
| PR001165 | 0.414 | 6457 | 99.66 |
| PR001166 | 0.204 | 7048 | 108.78 |
| PR001168 | 0.669 | 6776 | 104.58 |
| PR001170 | 0.352 | 6961 | 107.44 |
| PR001171 | 0.997 | 6597 | 101.82 |
| hRS7 | 0.753 | 6479 | 100 |

EC₅₀ and the median fluorescence intensity (MFI) maximum (MFIₘₐₓ) for the binding affinity of the Trop2 antibodies for the stably transfected strain expressing human Trop2 were ranked, and 29 Trop2 antibodies with EC₅₀ < 3 nM and MFIₘₐₓ > 4000 were selected for subsequent detection.

### Example 4: Determination of Binding Affinity and Dissociation Constant of Trop2 Antibodies for Recombinant Human or Monkey Trop2 Protein

Affinity was determined using an Octet RED96 instrument (Fortiebio) and an anti-human IgG Fc avidin sensor (AHC sensor, Pall ForteBio, #18-5060) according to the detailed procedures and methods provided by the manufacturer. Specifically, a human TROP2 protein (Sino Biological, #10428-H08H) or a monkey Trop2 protein (Sino Biological, #90893-C08H) was diluted to 200 nM with a PBS buffer (pH 7.4) containing 0.1% (w/w) BSA and 0.02% (v/v) tween 20, and incubated with the AHC sensor respectively. 40 nM Trop2 antibody was incubated at 30 °C for 3 min with the AHC sensor loaded with the human Trop2 protein or monkey Trop2 protein respectively. The reaction mixture was incubated at 30 °C for another 5 min in a PBS buffer (pH 7.4) containing 0.1% (v/w) BSA and 0.02% (v/v) Tween 20. The association and dissociation signals between the Trop2 antibody and the human Trop2 protein or monkey Trop2 protein were recorded by Octet Red 96 in real time. The affinity and the association and dissociation constants were determined by Octet using software. The results are shown in Tables 4, 5, and 6, with 4 of the 29 Trop2 antibodies tested (PR001128, PR001130, PR001166, PR001170) having KD values lower than those of the reference antibody Tab (hRS7) for both human Trop2 protein and monkey Trop2 protein, indicating their stronger binding affinity for human and monkey Trop2. In terms of binding affinity for the human Trop2 protein, in addition to the above four antibodies, PR001132, PR001138, PR001150, PR001159, PR001153, and PR001165 had KD values about 10-fold lower than that of the reference antibody; however, these antibodies had affinity for the monkey Trop2 protein comparable to that of the reference antibody.

**Table 4. Association and dissociation constants of Trop2 antibodies for human Trop2 protein**

| **Antigen** | **Antibody** | **Response** | **KD (M)** | **kon(1/Ms)** | **kdis(1/s)** |
|---|---|---|---|---|---|
| Human TROP2 | PR001128 | 0.4021 | <1.0E-12 | 1.30E+05 | <1.0E-07 |
| Human TROP2 | PR001130 | 0.3574 | <1.0E-12 | 5.52E+04 | <1.0E-07 |
| Human TROP2 | PR001132 | 0.4167 | <1.0E-12 | 1.21E+05 | <1.0E-07 |
| Human TROP2 | PR001133 | 0.3864 | 1.10E-09 | 8.85E+04 | 9.72E-05 |
| Human TROP2 | PR001134 | 0.354 | 5.42E-09 | 8.31E+04 | 4.51E-04 |
| Human TROP2 | PR001138 | 0.3381 | <1.0E-12 | 1.48E+05 | <1.0E-07 |
| Human TROP2 | PR001139 | 0.3362 | 4.79E-09 | 8.90E+04 | 4.26E-04 |
| Human TROP2 | PR001142 | 0.3898 | 3.23E-10 | 2.72E+05 | 8.78E-05 |
| Human TROP2 | PR001143 | 0.3346 | 6.94E-09 | 8.01E+04 | 5.56E-04 |
| Human TROP2 | PR001145 | 0.2897 | 1.47E-08 | 9.19E+04 | 1.35E-03 |
| Human TROP2 | PR001147 | 0.349 | 4.98E-10 | 8.50E+04 | 4.23E-05 |
| Human TROP2 | PR001150 | 0.3823 | 3.26E-11 | 1.61E+05 | 5.25E-06 |
| Human TROP2 | PR001151 | 0.4069 | 1.12E-09 | 1.13E+05 | 1.27E-04 |
| Human TROP2 | PR001152 | 0.3481 | 1.35E-10 | 1.54E+05 | 2.08E-05 |
| Human TROP2 | PR001153 | 0.3809 | 3.23E-11 | 7.86E+04 | 2.53E-06 |
| Human TROP2 | PR001154 | 0.4103 | 8.55E-10 | 1.08E+05 | 9.23E-05 |
| Human TROP2 | PR001155 | 0.4057 | 1.84E-09 | 7.81E+04 | 1.44E-04 |
| Human TROP2 | PR001156 | 0.3536 | 9.16E-11 | 1.83E+05 | 1.67E-05 |
| Human TROP2 | PR001158 | 0.433 | 1.55E-10 | 1.32E+05 | 2.05E-05 |
| Human TROP2 | PR001159 | 0.3802 | <1.0E-12 | 1.76E+05 | <1.0E-07 |
| Human TROP2 | PR001160 | 0.3903 | 8.19E-10 | 1.16E+05 | 9.48E-05 |
| Human TROP2 | PR001162 | 0.3661 | 1.70E-09 | 2.98E+05 | 5.07E-04 |
| Human TROP2 | PR001163 | 0.3956 | 7.90E-10 | 1.06E+05 | 8.34E-05 |
| Human TROP2 | PR001164 | 0.1942 | 2.41E-09 | 1.09E+05 | 2.62E-04 |
| Human TROP2 | PR001165 | 0.3798 | <1.0E-12 | 1.64E+05 | <1.0E-07 |
| Human TROP2 | PR001166 | 0.3237 | <1.0E-12 | 4.70E+05 | <1.0E-07 |
| Human TROP2 | PR001168 | 0.3325 | 1.59E-09 | 8.80E+04 | 1.40E-04 |
| Human TROP2 | PR001170 | 0.3683 | <1.0E-12 | 4.76E+05 | <1.0E-07 |
| Human TROP2 | PR001171 | 0.3257 | 2.51E-11 | 1.49E+05 | 3.74E-06 |
| Human TROP2 | Tab(hRS7) | 0.3492 | 1.99E-10 | 1.66E+05 | 3.30E-05 |

**Table 5. Association and dissociation constants of Trop2 antibodies for monkey Trop2 protein**

| **Antigen** | **Antibody** | **Response** | **KD (M)** | **kon(1/Ms)** | **kdis(1/s)** |
|---|---|---|---|---|---|
| Monkey Trop2 | PR001128 | 0.3835 | <1.0E-12 | 8.35E+04 | <1.0E-07 |
| Monkey Trop2 | PR001130 | 0.3145 | <1.0E-12 | 3.77E+04 | <1.0E-07 |
| Monkey Trop2 | PR001132 | 0.4181 | 1.64E-10 | 8.69E+04 | 1.43E-05 |
| Monkey Trop2 | PR001133 | 0.3691 | 2.49E-09 | 5.98E+04 | 1.49E-04 |
| Monkey Trop2 | PR001134 | 0.335 | 9.33E-09 | 5.49E+04 | 5.12E-04 |
| Monkey Trop2 | PR001138 | 0.3289 | 4.09E-10 | 9.74E+04 | 3.98E-05 |
| Monkey Trop2 | PR001139 | 0.3278 | 7.75E-09 | 6.81E+04 | 5.28E-04 |
| Monkey Trop2 | PR001142 | 0.3824 | 2.60E-10 | 1.65E+05 | 4.29E-05 |
| Monkey Trop2 | PR001143 | 0.3078 | 1.24E-08 | 5.21E+04 | 6.45E-04 |
| Monkey Trop2 | PR001145 | 0.2767 | 1.91E-08 | 7.01E+04 | 1.34E-03 |
| Monkey Trop2 | PR001147 | 0.3405 | 1.92E-09 | 6.09E+04 | 1.17E-04 |
| Monkey Trop2 | PR001150 | 0.3441 | 3.19E-10 | 1.07E+05 | 3.41E-05 |
| Monkey Trop2 | PR001151 | 0.3512 | 2.38E-09 | 7.58E+04 | 1.80E-04 |
| Monkey Trop2 | PR001152 | 0.3432 | 1.02E-09 | 1.03E+05 | 1.05E-04 |
| Monkey Trop2 | PR001153 | 0.3366 | 1.81E-10 | 5.93E+04 | 1.07E-05 |
| Monkey Trop2 | PR001154 | 0.3792 | 1.91E-09 | 7.27E+04 | 1.39E-04 |
| Monkey Trop2 | PR001155 | 0.3561 | 4.26E-09 | 5.15E+04 | 2.19E-04 |
| Monkey Trop2 | PR001156 | 0.3494 | 5.36E-10 | 1.15E+05 | 6.15E-05 |
| Monkey Trop2 | PR001158 | 0.4055 | 7.08E-10 | 8.79E+04 | 6.23E-05 |
| Monkey Trop2 | PR001159 | 0.3716 | 3.07E-10 | 1.11E+05 | 3.39E-05 |
| Monkey Trop2 | PR001160 | 0.3603 | 1.82E-09 | 7.43E+04 | 1.35E-04 |
| Monkey Trop2 | PR001162 | 0.3701 | 3.66E-09 | 2.00E+05 | 7.32E-04 |
| Monkey Trop2 | PR001163 | 0.3721 | 1.96E-09 | 6.84E+04 | 1.34E-04 |
| Monkey Trop2 | PR001164 | 0.173 | 2.04E-09 | 7.94E+04 | 1.62E-04 |
| Monkey Trop2 | PR001165 | 0.3925 | 2.60E-10 | 1.01E+05 | 2.62E-05 |
| Monkey Trop2 | PR001166 | 0.3224 | <1.0E-12 | 3.16E+05 | <1.0E-07 |
| Monkey Trop2 | PR001168 | 0.3111 | 2.45E-09 | 5.93E+04 | 1.45E-04 |
| Monkey Trop2 | PR001170 | 0.3865 | <1.0E-12 | 3.07E+05 | <1.0E-07 |
| Monkey Trop2 | PR001171 | 0.3194 | 1.51E-10 | 8.83E+04 | 1.34E-05 |
| Monkey Trop2 | Tab(hRS7) | 0.3224 | 1.37E-10 | 1.08E+05 | 1.48E-05 |

**Table 6. Comparison of dissociation constants of the antibodies for monkey Trop2 protein and human Trop2 protein**

| **Antibody** | **KD (M)** (antigen: monkey Trop2 protein) | **KD (M)** (antigen: human Trop2 protein) | **KD (monkey)/ KD (human)** |
|---|---|---|---|
| PR001128 | <1.0E-12 | <1.0E-12 | n/a |
| PR001130 | <1.0E-12 | <1.0E-12 | n/a |
| PR001132 | 1.64E-10 | <1.0E-12 | n/a |
| PR001133 | 2.49E-09 | 1.10E-09 | 2.27 |
| PR001134 | 9.33E-09 | 5.42E-09 | 1.72 |
| PR001138 | 4.09E-10 | <1.0E-12 | n/a |
| PR001139 | 7.75E-09 | 4.79E-09 | 1.62 |
| PR001142 | 2.60E-10 | 3.23E-10 | 0.81 |
| PR001143 | 1.24E-08 | 6.94E-09 | 1.78 |
| PR001145 | 1.91E-08 | 1.47E-08 | 1.3 |
| PR001147 | 1.92E-09 | 4.98E-10 | 3.86 |
| PR001150 | 3.19E-10 | 3.26E-11 | 9.76 |
| PR001151 | 2.38E-09 | 1.12E-09 | 2.12 |
| PR001152 | 1.02E-09 | 1.35E-10 | 7.57 |
| PR001153 | 1.81E-10 | 3.23E-11 | 5.61 |
| PR001154 | 1.91E-09 | 8.55E-10 | 2.24 |
| PR001155 | 4.26E-09 | 1.84E-09 | 2.32 |
| PR001156 | 5.36E-10 | 9.16E-11 | 5.85 |
| PR001158 | 7.08E-10 | 1.55E-10 | 4.56 |
| PR001159 | 3.07E-10 | <1.0E-12 | n/a |
| PR001160 | 1.82E-09 | 8.19E-10 | 2.22 |
| PR001162 | 3.66E-09 | 1.70E-09 | 2.16 |
| PR001163 | 1.96E-09 | 7.90E-10 | 2.48 |
| PR001164 | 2.04E-09 | 2.41E-09 | 0.85 |
| PR001165 | 2.60E-10 | <1.0E-12 | n/a |
| PR001166 | <1.0E-12 | <1.0E-12 | n/a |
| PR001168 | 2.45E-09 | 1.59E-09 | 1.54 |
| PR001170 | <1.0E-12 | <1.0E-12 | n/a |
| PR001171 | 1.51E-10 | 2.51E-11 | 6.02 |
| Tab | 1.37E-10 | 1.99E-10 | 0.69 |

### Example 5: Killing of Target Cells by Internalization of Antibodies Conjugated with MMAF

The Trop2 antibody can mediate cellular internalization of Trop2 proteins expressed on the surface by binding to the extracellular portion of Trop2. In this example, Trop2⁺ cells were tested for their susceptibility to killing by the Trop2 antibodies after the internalization of the Trop2 antibodies.

BxPC-3 cells were cultured and expanded in a T-75 flask in a DMEM medium (Life technologies, #11995-065) containing 10% serum (BI, fetal bovine serum, #04-002-1A). After reaching 90% fusion, the medium was removed by pipetting and the cells were washed twice with PBS. The cells were treated with trypsin (Invitrogen, #15050065) for about 1 min, and then the trypsin was neutralized with the medium. The cells were transferred to a 15 mL sterile centrifuge tube and centrifuged at room temperature for 5 min at 1000 rpm to obtain a cell mass. The medium was removed by pipetting, and the cells were resuspended in the corresponding medium. The cells were gently pipetted to obtain a single cell suspension. After counting with a cell counting plate, 2×10³ BxPC-3 cells were added to a black ViewPlate-96 TC (Perkin Elmer, #6005225) plate. The cells were incubated overnight in an incubator at 37 °C with 5% CO₂.

The next day, an antibody solution at 10× starting concentration (100 nM) was prepared with a FBS-free medium, and subjected to a 5-fold dilution to obtain 6 antibody concentrations. 10 µL of antibody sample at each gradient was transferred to the above cell plate, with a final volume of 100 µL in each well. 2 µL of 50 µg/mL αHFc-CL-MMAF medium (Moradec, αHFc-CL-MMAF kit, #AH-102AF) was added to each well to achieve a final concentration of 1 µg/mL. The cells were incubated at 37 °C with 5% CO₂ for 4 days.

On day 6, 100 µL of CellTiter-Glo^{®} luminescent cell activity reagent (Promega, USA, #G7570) was added to each well and mixed on a shaker for 2 min to induce cell lysis. The 96-well plate was incubated at room temperature for 10 min to stabilize the light signal. Luminescence was recorded using a PE Enspire microplate reader (Perkin Elmer, EnSpire). Viability = (1 - (IgG1_{mean luminescence value} - antibody sample_{luminescence value}) / IgG1_{mean luminescence value}) × 100%. The EC₅₀ value was determined from the viability.

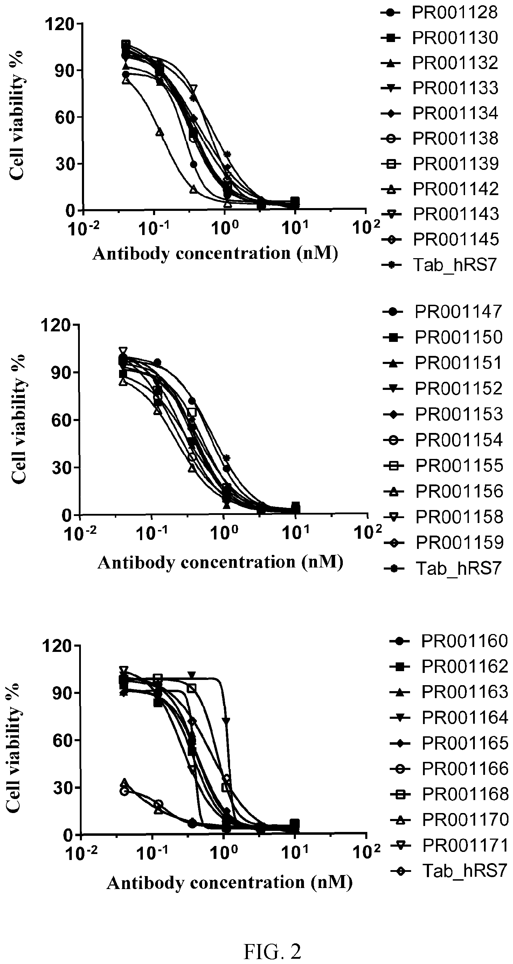

The viability of BxPC-3 cells under antibody treatment is shown in FIG. 2 and Table 7. The 27 antibodies of the present invention had EC₅₀ for internalization activity comparable to or better than that of hRS7, except for PR001163 and PR001164. Specifically, the EC₅₀ values of most of the Trop2 antibodies were lower than that of the reference antibody, indicating that they were capable of achieving the maximal antibody internalization killing effect at a lower concentration. Compared with the reference antibody Tab (hRS7), the highest cell viability under treatment of antibody PR001166 was significantly lower than the cell viability under Tab treatment, indicating that it had the best antibody internalization killing effect at a lower concentration.

**Table 7. Internalization killing activity of Trop2 antibody against target cell**

| Antibody No. | EC₅₀(nM) | Minimum cell viability % | Maximum cell viability % |
|---|---|---|---|
| PR001128 | 0.2812 | 5.059 | 87.77 |
| PR001130 | 0.346 | 2.622 | 103.2 |
| PR001132 | 0.3855 | 2.19 | 93.38 |
| PR001133 | 0.375 | 2.454 | 99.81 |
| PR001134 | 0.4174 | -1.342 | 111.7 |
| PR001138 | 0.3242 | 3.166 | 101.7 |
| PR001139 | 0.313 | 3.269 | 109.9 |
| PR001142 | 0.1314 | 3.344 | 91.76 |
| PR001143 | 0.6106 | 1.921 | 99.67 |
| PR001145 | 0.3823 | 2.107 | 107.3 |
| PR001147 | 0.6507 | 1.712 | 97.44 |
| PR001150 | 0.3282 | 2.32 | 91.45 |
| PR001151 | 0.2811 | 0.7009 | 103.5 |
| PR001152 | 0.4276 | 2.273 | 92.38 |
| PR001153 | 0.451 | 1.201 | 95.39 |
| PR001154 | 0.2118 | 2.462 | 107.9 |
| PR001155 | 0.5242 | 0.8184 | 92.29 |
| PR001156 | 0.2243 | 2.78 | 89.87 |
| PR001158 | 0.3612 | 0.5579 | 101.5 |
| PR001159 | 0.4044 | 1.934 | 95.9 |
| PR001160 | 0.4257 | 3.753 | 97.68 |
| PR001162 | 0.3922 | 3.221 | 92.52 |
| PR001163 | ∼ 0.3966 | 3.506 | 90.94 |
| PR001164 | ∼ 1.186 | 4.869 | 98.67 |
| PR001165 | 0.4608 | 2.824 | 91.61 |
| PR001166 | 0.147 | 4.528 | 28.47 |
| PR001168 | 0.8201 | 4.947 | 98.5 |
| PR001170 | 0.0213 | 3.396 | 91.97 |
| PR001171 | 0.276 | 2.063 | 105.9 |
| hRS7 | 0.709 | -1.22 | 100.9 |

According to the EC₅₀ for the binding of the antibodies to HEK293/hTrop2, the germline diversity of the antibody sequences and the number of post-translational modification sites (Table 8), the affinity for a human or monkey protein, and the superiority of the endocytic effect, 8 antibodies in Table 8 were selected for subsequent analysis.

**Table 8. Germline and post-translational modification sites of sequences of Trop2 antibody**

| Antibody No. | Germline of heavy chain | Germline of light chain | Post-translational modification of heavy chain | Post-translational modification of light chain |
|---|---|---|---|---|
| PR001128 | VH4_4*02 | Vk3_11^{∗}01 | | |
| PR001130 | VH4_4*02 | Vk1_5^{∗}01 | | |
| PR001133 | VH3_30^{∗}18 | Vk1_39^{∗}01 | DG (HCDR2), DG (HFR3) | NS (LCDR1) |
| PR001139 | VH3_30*01 | Vk3_15*01 | DG (HCDR2) | |
| PR001142 | VH4_4*02 | Vk1_5^{∗}01 | | |
| PR001145 | VH3_30*01 | Vk3_15*01 | DG (HCDR2) | |
| PR001162 | VH3_30*01 | Vk3_11^{∗}01 | DG (HCDR1), DG (HCDR2) | |
| PR001165 | VH3_30*01 | Vk1_9^{∗}01 | DG (HCDR2), DG (HFR3) | NS (LCDR3) |
| PR001166 | VH4_38-2*01 | Vk3_15*01 | | |

### Example 6: Binding of Recombinant Antibodies to BxPC-3 Tumor Cell Line

BxPC-3 tumor cells (the Cell Bank of the Chinese Academy of Sciences, #TCHu 12) were digested with 3 mL of TrypLE, and the digestion was stopped with 7 mL of the corresponding medium. Cell density was determined, and 1×10⁵ cells were collected for the test sample in each well. The cell solution was centrifuged, and the cells were washed once with PBS + 0.5% BSA buffer. The cells were suspended in 100 µL of antibody diluted in a gradient (5 fold dilution, starting concentration: 100 nM) and incubated at 4 °C for 1 h, with hRS7 and hIgG1 as controls. The cells were washed once with PBS + 0.5% BSA. The cells were incubated at 4 °C for 1 h in the dark with 100 µL of Alexa Fluor 488 goat anti-human IgG (Life technologies, #A-11013) diluted at 1:1000, and washed twice with PBS + 0.5% BSA. The cells were resuspended in 200 µL of PBS + 0.5% BSA. The suspension was centrifuged at 4 °C for 5 min at 2000 rpm and tested using a FACS verse flow cytometer or NovoCyte flow cytometer (ACEA Biosciences). All procedures were performed on ice.

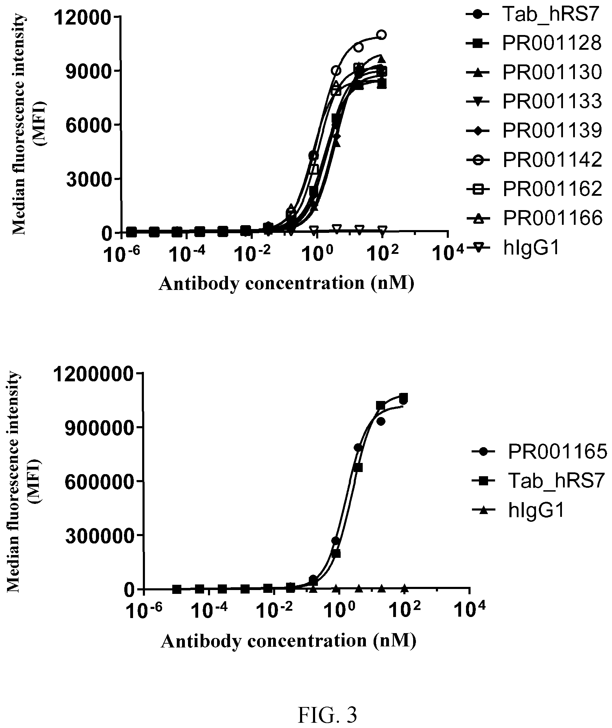

The binding of the antibody to BxPC tumor cells is shown in FIG. 3 and Table 9. Compared with the control hRS7, these 8 antibodies of the present invention showed the binding ability to BxPC tumor cells comparable to or higher than that of the reference antibody. The 3 antibodies of the present invention (PR001142, PR001162, and PR001166) had lower EC₅₀ than that of the reference antibody, with EC₅₀ about two times less than EC₅₀ of the reference antibody, indicating that these antibodies were capable of binding to Trop2 more sensitively at a lower concentration.

**Table 9. Binding ability of Trop2 antibody to BxPC-3 tumor cells**

| | BxPC-3 | |
|---|---|---|
| Antibody No. | EC₅₀ (nM) | Maximum MFl value |
| PR001128 | 1.704 | 8412 |
| PR001130 | 3.852 | 9962 |
| PR001133 | 2.071 | 8948 |
| PR001139 | 3.145 | 9361 |
| PR001142 | 1.146 | 10849 |
| PR001162 | 1.117 | 9107 |
| PR001166 | 0.7157 | 8393 |
| hRS7 | 2.084 | 8727 |
| hlgG1 | / | 77 |
| PR001165 | 1.707 | 1012414 |
| Tab(hRS7) | 2.67 | 1078897 |
| hIgG1 | / | 670 |

### Example 7: Binding of Recombinant Antibodies to CHO-Kl/Monkey Trop2

CHO-Kl/monkey Trop2 cells (Chempartner,Shanghai, China) and CHO-K1 blast cells were each digested with 3 mL of TrypLE, and the digestion was stopped with 7 mL of the corresponding medium. Cell density was determined, and 2×10⁵ cells were collected for the test sample in each well. The cell solution was centrifuged, and the cells were washed once with PBS + 0.5% BSA. The cells were suspended in 100 µL of antibody diluted in a gradient (5 fold dilution, starting concentration: 100 nM) and incubated at 4 °C for 1 h, with hRS7 and hIgG1 as controls. The cells were washed once with PBS + 0.5% BSA. The cells were incubated at 4 °C for 1 h in the dark with 100 µL of Alexa Fluor 488 goat anti-human IgG (Life technologies, #A-11013) diluted at 1:1000, and washed twice with PBS + 0.5% BSA. The cells were resuspended in 200 µL of PBS + 0.5% BSA. The suspension was centrifuged at 4 °C for 5 min at 2000 rpm and tested using a FACS verse flow cytometer or NovoCyte flow cytometer (ACEA Biosciences). All procedures were performed on ice.

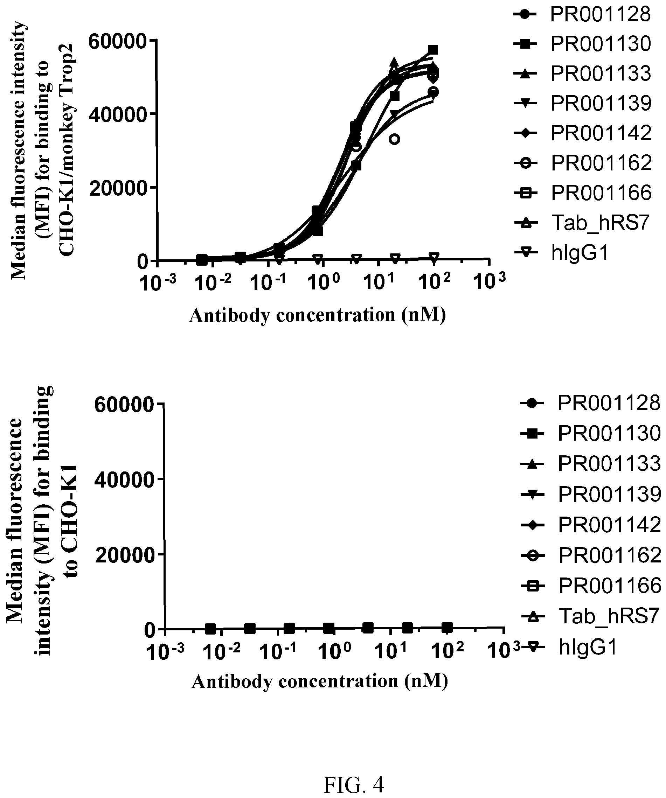

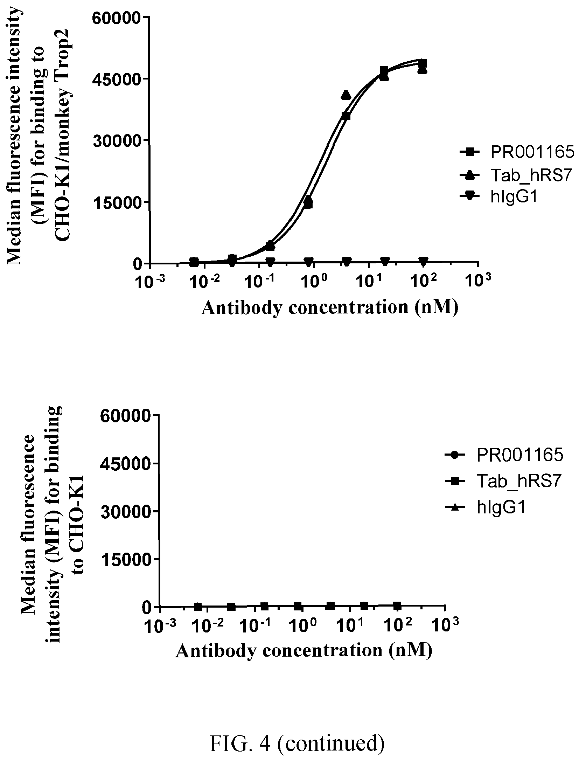

As shown in FIG. 4 and Table 10, the 8 antibodies of the present invention had binding ability to monkey Trop2 comparable to that of the reference antibody hRS7, and among them, PR001166 had EC₅₀ lower than EC₅₀ of the reference antibody, indicating that the antibody was capable of binding to Trop2 more sensitively at a lower concentration. In contrast, PR001130 had slightly weak binding ability to monkey Trop2.

**Table 10. Binding ability of Trop2 antibody to CHO-Kl/monkey Trop2 and CHO-K1**

| Antibody No. | CHO-K1/monkey Trop2 | | CHO-K1 | |
|---|---|---|---|---|
| | EC₅₀ (nM) | Maximum MFI value | EC₅₀ (nM) | Maximum MFl value |
| PR001128 | 2.573 | 53221 | / | 104.5 |
| PR001130 | 6.256 | 61693 | / | 104.5 |
| PR001133 | 2.866 | 55333 | / | 105.9 |
| PR001139 | 3.329 | 46430 | / | 104.5 |
| PR001142 | 2.101 | 51218 | / | 104.5 |
| PR001162 | 2.245 | 45496 | / | 104.5 |
| PR001166 | 1.97 | 51463 | / | 105.9 |
| Tab_hRS7 | 2.134 | 53057 | / | 107.2 |
| hlgG1 | ∼ 1894 | 277.4 | / | 105.9 |
| | | | | |
| PR001165 | 1.8 | 50254 | / | 101.4 |
| Tab_hRS7 | 1.302 | 49024 | / | 100 |
| hlgG 1 | / | 109.4 | / | 98.6 |

### Example 8: Binding of Recombinant Antibodies to Mouse Trop2

An ELISA plate was coated with 100 µL of 1 µg/mL mouse Trop2-his protein (Sino Biological, #50922-M08H) in PBS at 4 °C overnight. The plate was blocked with 200 µL of PBST (PBS + 0.05% tween 20) + 2% BSA at 37 °C for 1 h. The plate was washed 4 times with PBST. 100 µL of Trop2 antibody diluted in a gradient (5-fold dilution, starting concentration: 33.33 nM) was added to the ELISA plate and the plate was incubated at 37 °C for 1 h. The plate was washed 4 times with PBST. 100 µL of anti-human IgG (H+L)-HRP (Sigma, #A8667) diluted in 1:4000 was added and the plate was incubated at 37 °C for 1 h. The plate was washed 4 times with PBST. 100 µL of TMB substrate (Invitrogen, #88-7025-88) was added and the plate was incubated at room temperature for 5 min, and then 50 µL of stop buffer (BBI life sciences, #E661006-0200) was added to stop the reaction. The OD450 nm reading was recorded using a microplate reader (Perkin Elmer, EnSpire).

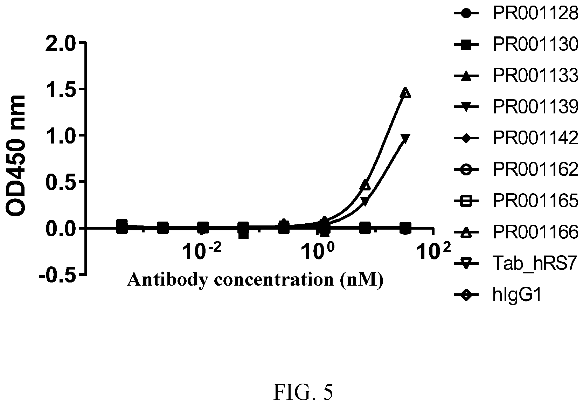

The results in FIG. 5 showed that the antibodies PR001139 and PR001166 bound to the mouse Trop2 protein, and the reference antibody hRS7 and other 6 Trop2 antibodies did not have mouse Trop2 binding activity.

Three antibodies PR001142, PR001165, and PR001166 were selected according to the results of the above experiments and further tested for antibody-dependent cell-mediated cytotoxicity, complement-dependent cytotoxicity, and antibody-dependent cell-mediated phagocytosis.

### Example 9: ADCC Activity Mediated by Trop2 Antibodies

Since the intracellular lactate dehydrogenase (LDH) is increased after cell lysis, the killing effect on tumor cells can be determined by detecting the amount of LDH released. The antibody was first diluted to 40 nM with RPMI 1640 containing 2% FBS, and then subjected to a 5-fold dilution from 40 nM to obtain 7 different concentrations. 25 µL of the antibody dilution was made to a final volume of 100 µL to obtain 7 concentrations of 10.0 nM, 2.0 nM, 0.4 nM, 0.08 nM, 0.016 nM, 0.0032 nM, and 0.00064 nM. BxPC-3 and peripheral blood mononuclear cells (PBMCs, Miaotong, #PB050F) were each collected and centrifuged at 1200 rpm for 5 min. The supernatant was discarded, and the cells were resuspended in a phenol red-free RPMI-1640 medium containing 2% FBS. PBMCs were adjusted to a density of 4×10⁶ cells/mL and added to a 96-well U-bottom plate (Corning, #3799) at 50 µL/well; and BxPC-3 cells were adjusted to a density of 4×10⁵ cells/mL and added to the same 96-well plate at 25 µL/well, with an effector-to-target ratio of 20:1 and three replicates set. 25 µL of the corresponding diluted antibody (ER: BxPC-3 + PBMC + mAb) was added to the same plate and the plate was incubated in a carbon dioxide incubator at 37 °C for 4 h. Different control groups were also set in the plate: ER (0): BxPC-3 + PBMC + 2% FBS + 1640; ESR: PBMC + 2% FBS + 1640; TSR: BxPC-3 + 2% FBS + 1640; CMB: 2% FBS + 1640; TMR: BxPC-3 + 2% FBS + 1640 + lysis buffer (lysis buffer was added after 3 h of incubation); VCC: 2% FBS 1640+ lysis buffer (lysis buffer was added after 3 h of incubation). After the incubation was completed, 50 µL of the supernatant was taken and added to a 96 well plate (Corning, #3599), and 50 µL of cytotoxicity assay reagent (CytoTox 96^{®} non-radioactive cytotoxicity assay kit, Promega, USA, #G1780) was added. The mixture was incubated at room temperature for 30 min, and then the stop solution was added to stop the reaction and the OD490 value was read using Enspire (Perkin Elmer). This reading was used to calculate the ADCC effect according to the following formula: ADCC specific killing % = {(ER - CMB) - [(ESR - CMB) + (TSR - CMB)]} / (TMR - VCC) × 100%. The process was repeated for 2 different PBMC donors (Lot#1152 and Lot#2153). The maximum specific killing % and EC₅₀ values are summarized in Table 11.

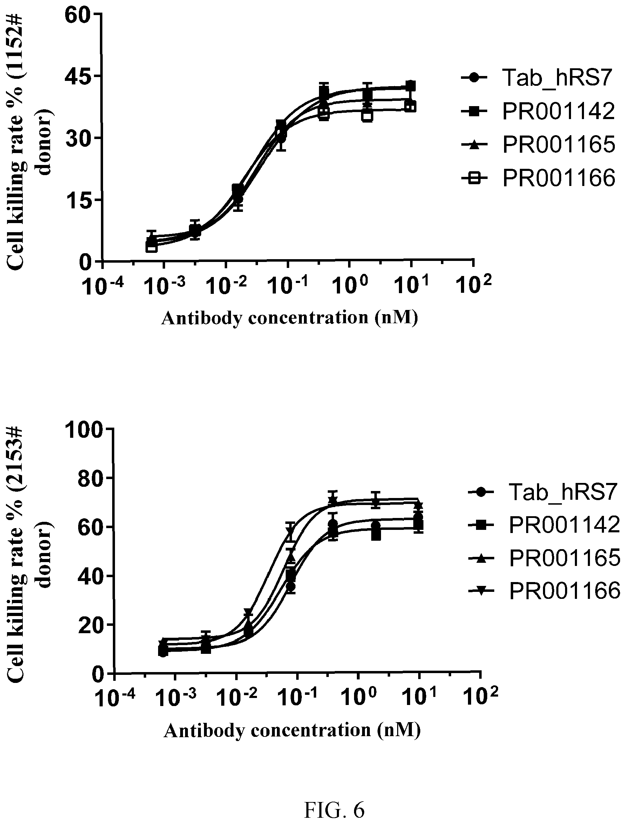

FIG. 6 showed the killing effect on BxPC-3 cells mediated by 3 Trop2 antibodies in PBMCs from different donors. As can be seen from Table 11, the 3 antibodies all showed specific killing of BxPC-3 cells. In the case of using PBMCs from two different donors 1152# and 2153#, in terms of the mean and standard error of specific killing rate, the antibody PR001166 had maximum killing rate comparable to that of the reference antibody Tab (hRS7), but had a lower EC₅₀ value, indicating that the antibody PR001166 was capable of achieving killing comparable to that of the reference antibody at a lower concentration. In contrast, the other two antibodies PR001142 and PR001165 had comparable killing to that of the reference antibody.

**Table 11. ADCC effect of 3 Trop2 antibodies on BxPC-3**

| Antibody No. | ADCC (donor 1152#) | | | ADCC (donor 2153#) | | |
|---|---|---|---|---|---|---|
| | EC₅₀(nM) | Maximum cell killing % | Minimum cell killing % | EC₅₀(nM) | Maximum cell killing % | Minimum cell killing % |
| Tab(hRS7) | 0.03907 | 42.06 | 4.321 | 0.08005 | 62.65 | 10.18 |
| PR001142 | 0.02791 | 41.58 | 4.47 | 0.05466 | 58.79 | 9.276 |
| PR001165 | 0.03236 | 38.96 | 5.793 | 0.06155 | 70.79 | 14.02 |
| PR001166 | 0.01999 | 36.48 | 3.255 | 0.03469 | 69.1 | 11.9 |

### Example 10: ADCP Activity of Trop2 Antibodies

Antibody-dependent cell-mediated phagocytosis is an important mechanism of action of therapeutic antibodies against viral infections or neoplastic diseases.

CD14⁺ monocytes were isolated from PBMCs (Miaotong, #PB050F) using human CD14 sorting beads (Meltenyi Biotech, #130-050-201) and resuspended at a density of 1×10⁶ cells/mL in an RPMI1640 medium containing 10% FBS, and GM-CSF (PeproTech, #300-03-A) was added at 100 ng/mL. Monocytes were seeded in a 6-well plate at 2×10⁶ cells/well and incubated in a carbon dioxide incubator at 37 °C for 9 days to differentiate into macrophages. The medium (containing 100 ng/mL GM-CSF) was changed every 3-4 days. After 9 days, macrophages were digested with trypsin, and the trypsin reaction was stopped with RPMI1640 containing 10% FBS. The cells were collected, washed once with PBS, and resuspended in PBS to obtain a density of 1×10⁶ cells/mL. BxPC-3 cells were also collected and resuspended in PBS to obtain a density of 1×10⁶ cells/mL. Macrophages were stained with 0.1 µM Far-red (in PBS), and BxPC-3 cells were stained with 0.5 µM CFSE (in PBS) at 4 °C for 10 min. The stained cells were centrifuged and washed once with more than 20 mL of RPMI1640 + 10% FBS medium. The washed cells were resuspended in 1% BSA + RPMI1640 medium and adjusted to a cell density of 1.6×10⁶ cells/mL. To a 96-well V-bottom plate (Corning, #3894) were added BxPC-3 cells at 25 µL/well (4×10⁴ cells/well) and macrophages at 25 µL/well (4×10⁴ cells/well). The antibody was diluted with 1% BSA + RPMI1640 to an intermediate concentration of 20 nM and further subjected to a 5-fold dilution from 20 nM to obtain 7 gradients. The diluted antibody was added at 50 µL/well to the same 96-well V-bottom plate containing BxPC-3 and macrophages. The mixture was mixed well and incubated at 37 °C for 1 h. The proportion of FITC + BxPC-3 cells and Alexa647 + macrophages that were double positive was identified by flow cytometry using a NovoCyte flow cytometer (ACEA Biosciences). Data were analyzed using FlowJo software (Tree Star, Ashland, OR), and the percentage of double-stained cells was used to determine ADCP-mediated phagocytosis.

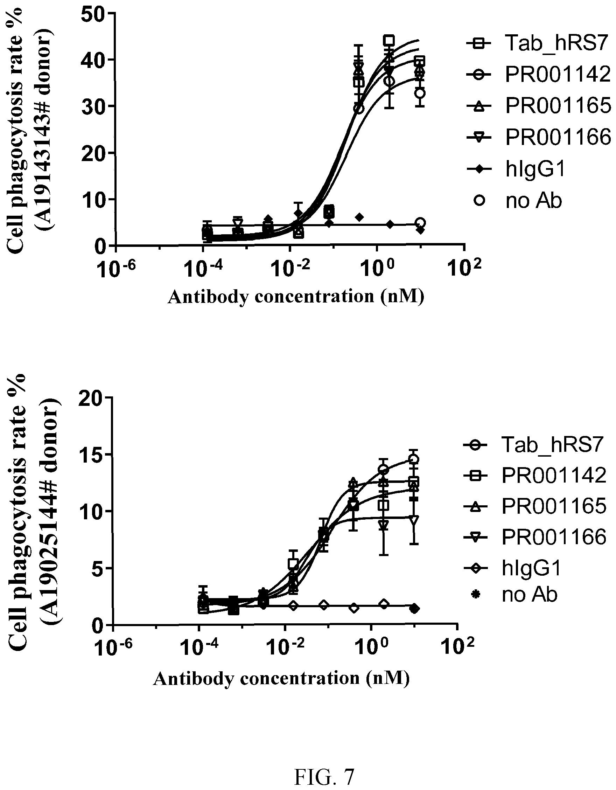

FIG. 7 showed the phagocytosis of BxPC-3 mediated by 3 Trop2 antibodies in macrophages from different donors. As shown in Table 12, the 3 antibodies all showed the ADCP effect on BxPC-3 cells. In the case of using PBMCs from donor A1925144#, in terms of the mean and standard error of specific killing, the 3 antibodies all had the maximum phagocytosis % close to that of the reference antibody Tab, but had EC₅₀ values less than that of Tab, indicating that they were capable of achieving maximum killing comparable to or higher than that of the reference antibody at a lower concentration. However, in the case of using PBMCs from donor A19143143#, in terms of the mean and standard error of specific killing, the 3 antibodies had the maximum phagocytosis % that was not significantly different from that of Tab, but had a lower EC₅₀ value, indicating that they were capable of achieving maximum killing comparable to or higher than that of the reference antibody at a lower concentration. Both donors showed that these 3 antibodies were capable of achieving killing comparable to that of the reference antibody at a lower concentration.

**Table 12. ADCP Effect of 3 Trop2 antibodies on BxPC-3**

| Antibody No. | ADCP (Donor A19143143#) | | | ADCP (Donor A1925144#) | | |
|---|---|---|---|---|---|---|
| | EC₅₀(nM) | Maximum phagocytosis % | Minimum phagocytosis % | EC₅₀(nM) | Maximum phagocytosis % | Minimum phagocytosis % |
| Tab(hRS7) | 0.2083 | 44.83 | 1.041 | 0.1247 | 14.83 | 1.721 |
| PR001142 | 0.1994 | 36.4 | 1.754 | 0.03618 | 11.98 | 0.861 |
| PR001165 | 0.1777 | 42.68 | 1.37 | 0.06833 | 12.52 | 2.245 |
| PR001166 | 0.1609 | 40.25 | 2.045 | 0.03106 | 9.333 | 2.048 |

### Example 11: CDC Activity of Trop2 Antibodies

The CDC activity of Trop2 antibody was evaluated by a complement-mediated cell killing assay. BxPC-3 cells or HEK293/hTrop2 cells were digested with trypsin. After the trypsin reaction was stopped, BxPC-3 cells or HEK293/hTrop2 cells were resuspended in serum-free RPMI-1640, and 1×10⁴ BxPC-3 cells were seeded into a black transparent-bottom 96-well plate (ViewPlate-96 TC, black, Perkin Elmer, #6005225) at 25 µL/well. The antibody was diluted with serum-free RPMI-1640, and the antibodies at different gradients were added at 25 µL/well. 50 µL of human serum was added to each well, and the mixture was mixed well and incubated in a carbon dioxide incubator at 37 °C for 24 h. Cell viability was tested using the Celltiter Glo luminescent cell viability kit (Promega, #G7573) according to the method provided by the manufacturer. The culture dish was shaken on a microplate shaker at 200 rpm for 2 min and then incubated at room temperature for 10 min. The luminescence signal was read with a plate reader (Perkin Elmer, EnSpire). For data analysis, the percentage of cytotoxicity was calculated using graphpad prism 7.0.

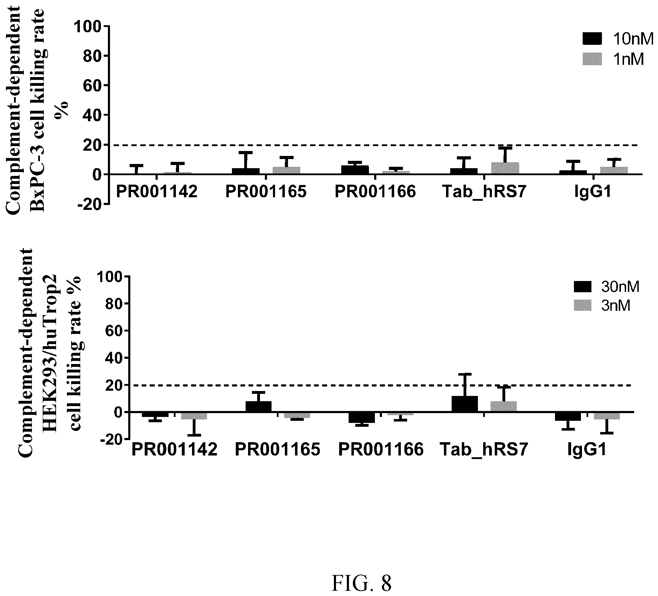

The results in FIG. 8 showed that the 3 antibodies and the reference antibody Tab (hRS7) all did not show CDC activity on BxPC-3 and HEK293/hTrop2 cells (CDC < 20%).

### Example 12: Binding Affinity of Antibodies for Human and Monkey Trop2 Proteins Tested by BIACORE

HBS-EP+ (10 mM HEPES, 150 mM NaCl, 3 mM EDTA, and 0.05% P20, pH 7.4, GE Healthcare, BR-1001-88) was used as a running buffer throughout the test. The antigen human Trop2 protein or monkey Trop2 protein was immobilized on the surface of a chip in a manual operation mode by the following procedures: 1) flow cell 3 of a series S CM5 sensor chip (GE Healthcare, #29-1275-56) was activated with a fresh mixture of 50 mM *N*-hydroxysuccinimide (NHS) and 200 mM 1-ethyl-3-(3-dimethylaminopropyl)carbodiimide hydrochloride (EDC) at 10 µL/min for 420 s; 2) 0.2 µg/mL human Trop2 or monkey Trop2 diluted in 10 mM NaOAc (pH 5.0) was injected into the activation flow cell 3 at 10 µL/min for 120 s; and 3) the remaining active ester groups were blocked with 1 M ethanolamine (pH 8.5) at 10 µL/min for 120 s. Then, the diluted antibodies at different concentrations (at concentrations of 0 nM, 3.125 nM, 6.25 nM, 12.5 nM, 25.0 nM, 50.0 nM, and 100 nM, respectively) were injected into flow cells 1, 2, 3, and 4 at 30 µL/min, and the association time was set to 180 s. The dissociation time for PR001142, PR001165, and hRS7 was 900 s, and the dissociation time for PR001166 was 1800 s. To remove the test antibodies from the surface, 10 mM glycine-HCl, pH 1.5 (GE Life Sciences, #BR-1003-54) was injected into flow cells 1, 2, 3, and 4 at 30 µL/min for 30 s. Data were analyzed using Biacore T200 (GE Healthcare, T200 model, #BIAC-B20-03) Evaluation 3.1 software.

As shown in Table 13, BIACORE test results showed that in terms of the binding affinity for either the human Trop2 protein or the monkey Trop2 protein, the antibodies PR001142 and PR001165 had binding affinity comparable to that of hRS7, while PR001166 had binding affinity higher than that of hRS7.

**Table 13. Binding affinity of Trop2 antibodies for human and monkey Trop2 proteins determined by BIACORE**

| Antibody No. | Human Trop2 protein antigen (His tag) | | | Monkey Trop2 protein antigen (His tag) | | |
|---|---|---|---|---|---|---|
| | ka (1/Ms) | kd (1/s) | KD (M) | ka (1/Ms) | kd (1/s) | KD (M) |
| PR001142 | 4.75E+05 | 2.52E-04 | 5.30E-10 | 4.79E+05 | 1.79E-04 | 3.74E-10 |
| PR001165 | 3.07E+05 | 1.52E-04 | 4.95E-10 | 2.55E+05 | 1.25E-04 | 4.91E-10 |
| PR001166 | 2.07E+06 | 5.78E-05 | 2.79E-11 | 1.59E+06 | 1.37E-04 | 8.56E-11 |
| hRS7 | 3.24E+05 | 1.94E-04 | 6.01E-10 | 3.64E+05 | 2.25E-04 | 6.19E-10 |

### Example 13: Epitope Binning of Antigen-Binding Proteins

The resulting antigen-binding proteins (PR001142, PR001165, and PR001166) and hRS7 were subjected to epitope binning on the ForteBio Octet platform. The biotin molecule (Sulfo-NHS-LC-Biotin, Thermo Fisher, #A39257) was incubated with a human Trop2 protein (Sino biological, #10428-H08H) according to a molar ratio of 3:1 at room temperature for 2 h. The biotinylated mixture after the incubation was washed three times with PBS and a Zeba centrifugal desalting column (Thermo Fisher, #89882) to remove excess biotin. The biotinylated Trop2 protein was captured by an SA sensor (Pall ForteBio, #18-5019) with the loading baseline set at 0.3 nm. The sensor was immersed in a well of assay buffer containing 50 nM primary antibody for 300 s. The sensor was then transferred to a well of assay buffer containing 50 nM primary antibody for 300 s. If the secondary antibody exhibited significant binding, it was considered a non-competitor (i.e., in an epitope interval different from that of the primary antibody). If the secondary antibody exhibited no significant binding, it was considered a competitor (i.e., in the same epitope interval as the primary antibody). The binding assay was performed by comparing the binding of the secondary antibody to Trop2 in the presence of the primary antibody to the blocking of the primary antibody itself.

As shown in Table 14 below, the results showed that the Trop2-binding sites of PR001142 and PR001165 overlapped with those of hRS7 to a high degree, while the Trop2-binding sites of PR001166 overlapped with those of hRS7 and PR001165 to a low degree.

**Table 14. Epitope binning of the antigen-binding proteins of the present application and clinical antibodies**

| Inhibition rate (%) | | Secondary antibody | | | |
|---|---|---|---|---|---|
| | | PR001142 | PR001165 | PR001166 | hRS7 |
| Primary antibody | PR001142 | 100.7 | 99.19 | 85.51 | 97.78 |
| | PR001165 | 98.08 | 97.73 | 5.24 | 96.44 |
| | PR001166 | 85.4 | 18.5 | 101.7 | 21.82 |
| | hRS7 | 93.27 | 92.04 | 7.34 | 96.49 |

### Example 14: Short-time Endocytic Effect of Trop2 Antibodies on Target Cells

The Trop2 antibody can mediate internalization of Trop2 proteins expressed on the cell surface by binding to the extracellular portion of Trop2. In this example, the Trop2 antibody was tested for internalization at different time points over a short period of time (within 3 h).

BxPC-3 or HEK293/hTrop2 cells were cultured and expanded in a T-75 flask in a DMEM medium (Life technologies, #11995-065) containing 10% serum (BI, fetal bovine serum, #04-002-1A). After reaching 90% fusion, the medium was removed by pipetting and the cells were washed twice with PBS. The cells were treated with trypsin (Invitrogen, #15050065) for about 1 min, and then the trypsin was neutralized with the medium. The cells were transferred to a 15 mL sterile centrifuge tube and centrifuged at room temperature for 5 min at 1000 rpm to obtain a cell mass. The medium was removed by pipetting, and the cells were resuspended in the corresponding medium. The cells were gently pipetted to obtain a single cell suspension. After counting with a cell counting plate, BxPC-3 cells were resuspended to 2×10⁶ cells/mL in an ice-cold FACS buffer (PBS + 2% FBS), and the cell suspension was added to a 96-well V-bottom plate (Corning, #3894) at 100 µL/well and centrifuged at 400 g for 5 min. After the supernatant was discarded, for the assay without endocytosis, the following procedures were performed: 100 µL of FACS buffer containing PR001142 or PR001165 or PR001166 or hIgG1 at a final concentration of 100 nM was added and the plate was incubated at 4 °C for one hour. The cells were washed twice with an ice-cold FACS buffer to remove unbound antibodies. For the assay with endocytosis, the cells added with the antibody were left to stand at 37 °C for 0/15 min/30 min/45 min/1 hr/1.5 hr/2 hr/3 hr, and then the cells were transferred to 4 °C, and pre-cooled PBS was added to prevent the endocytosis of the antibody. The cells were washed twice with an ice-cold FACS buffer to remove unbound antibodies. In both cases, the cells were left to stand using 2 µg/mL Alexa Fluor 488 goat anti-human IgG (H+L) (Jackson ImmunoResearch, #109-545-003) in an ice-cold FACS buffer at 4 °C for half an hour. The cells were washed twice with an ice-cold FACS buffer to remove unbound secondary antibodies. The cells were resuspended in 200 µL of FACS buffer. The fluorescence intensity of the cells was analyzed using a FACS canto II flow cytometer. Endocytosis rate % = (1 - MFI_{37 °C} / MFI_{4 °C}) × 100%.

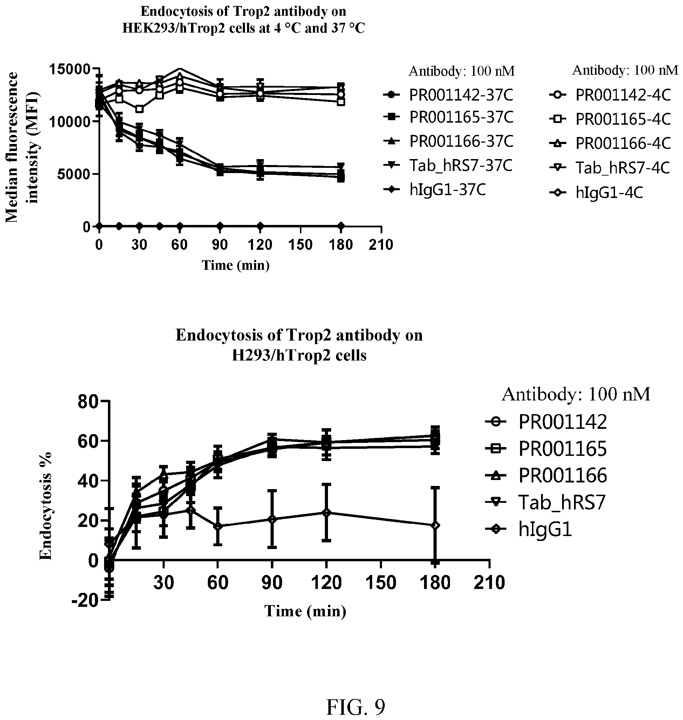

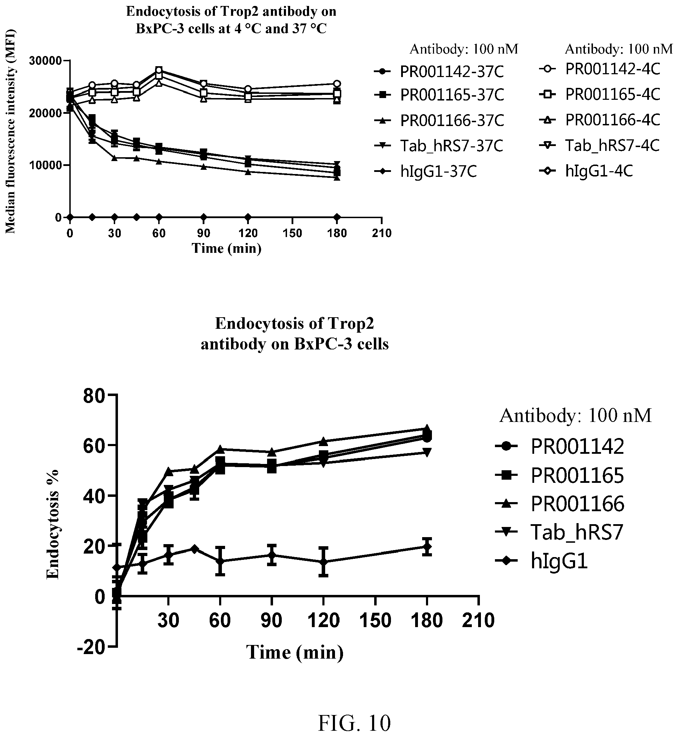

As shown in FIGs. 9 and 10, the results showed that the endocytosis rates of PR001142/PR001165/PR001166 at saturating antibody-binding concentrations in the recombinant cell line HEK293/hTrop2 or in Trop2-positive tumor cells BxPC-3 were not significantly different from each other. Within 1 hour, however, PR001166 had a better endocytic effect than that of other antibodies, including the reference antibody.

All documents mentioned in the present invention are incorporated by reference in this application as if each were individually incorporated by reference. Furthermore, it should be understood that various changes or modifications of the present invention can be made by those skilled in the art after reading the above teachings of the present invention, and these equivalents also fall within the scope of the appended claims of the present application.

## Claims

1. An antibody or an antigen-binding fragment against Trop-2 comprising complementarity determining regions (CDRs) as follows:
(a) an HCDR1 or a variant of a sequence thereof, an HCDR2 or a variant of a sequence thereof, and an HCDR3 or a variant of a sequence thereof comprised in a heavy chain variable region (VH) set forth in any one of SEQ ID NOs: 217, 199, 216, 213, 192, 193, 195, or 198; and/or
(a) an LCDR1 or a variant of a sequence thereof, an LCDR2 or a variant of a sequence thereof, and an LCDR3 or a variant of a sequence thereof comprised in a light chain variable region (VL) set forth in any one of SEQ ID NOs: 238, 229, 235, 236, 221, 222, 225, or 228;
preferably, the variant of the sequence is a CDR having one or several amino acid substitutions, deletions, or additions compared to a CDR from which the variant is derived; preferably, the substitutions are conservative substitutions.

2. The antibody or the antigen-binding protein according to claim 1, wherein the antibody or the antigen-binding protein is one of antibodies having combinations of heavy chain VH-CDR1, VH-CDR2, and VH-CDR3, and light chain VL-CDR1, VL-CDR2, and VL-CDR3 as shown in the table below:
| **Antibody No.** | **Name of antibody** | **LCDR1** | **LCDR2** | **LCDR3** | **HCDR1** | **HCDR2** | **HCDR3** |
|---|---|---|---|---|---|---|---|
| (27) | PR001166 | 131 | 153 | 184 | 23 | 59 | 100 |
| (9) | PR001142 | 123 | 145 | 177 | 25 | 54 | 87 |
| (26) | PR0011 65 | 127 | 150 | 180 | 27 | 55 | 94 |
| (23) | PR001162 | 129 | 144 | 182 | 28 | 57 | 98 |
| (1) | PR001128 | 115 | 144 | 169 | 19 | 47 | 86 |
| (2) | PR001130 | 116 | 145 | 170 | 20 | 48 | 87 |
| (5) | PR001133 | 119 | 148 | 173 | 21 | 50 | 89 |
| (8) | PR001139 | 122 | 149 | 176 | 24 | 53 | 92 |
wherein, any one of the amino acid sequences described above further comprises a derived sequence optionally subjected to addition, deletion, modification, and/or substitution of at least one amino acid and capable of retaining the binding affinity for TROP2.

3. The antibody or the antigen-binding fragment according to claim 1 or 2, wherein the antibody or the antigen-binding fragment thereof is selected from an scFv, a Fab, a Fab', a (Fab')₂, an Fv fragment, a disulfide-linked Fv (dsFv), and a diabody.

4. A recombinant protein comprising:
(i) the antibody or the antigen-binding protein according to claim 1; and
(ii) an optional tag sequence to assist in expression and/or purification.

5. A polynucleotide encoding a polypeptide selected from the group consisting of: the antibody or the antigen-binding protein according to any one of claims 1 to 3, and/or the recombinant protein according to claim 4.

6. A vector comprising the polynucleotide according to claim 5.

7. A genetically engineered host cell comprising the vector according to claim 6 or the polynucleotide according to claim 5 integrated in the genome thereof.

8. A method for preparing the antibody or the antigen-binding fragment thereof according to any one of claims 1 to 3, comprising culturing the host cell according to claim 7 under conditions allowing expression of the antibody or the antigen-binding fragment thereof, and isolating the antibody or the antigen-binding fragment thereof from a cultured host cell culture.

9. An antibody-drug conjugate comprising:
(a) an antibody moiety comprising the antibody or the antigen-binding protein according to claims 1 to 3; and
(b) a conjugate moiety conjugated with the antibody moiety, wherein the conjugate moiety is selected from the group consisting of: a detectable label, a drug, a toxin, a cytokine, a radionuclide, an enzyme, and combinations thereof;
wherein the antibody moiety is conjugated with the conjugate moiety through a chemical bond or a linker.

10. A chimeric antigen receptor (CAR) comprising the antibody or the antigen-binding protein according to claims 1 to 3.

11. An immune cell expressing the chimeric antigen receptor (CAR) according to claim 10.

12. A multispecific antibody comprising the antibody or the antigen-binding fragment against Trop-2 according to any one of claims 1 to 3, and an additional antibody or a fragment or an antibody analog thereof; preferably, the multispecific antibody is bispecific, trispecific, or tetraspecific.

13. A pharmaceutical composition comprising:
(i) an active ingredient selected from the group consisting of: the antibody or the antigen-binding protein according to claims 1 to 3, the recombinant protein according to claim 4, the antibody-drug conjugate according to claim 9, the immune cell according to claim 11, the multispecific antibody according to claim 12, and combinations thereof; and
(ii) a pharmaceutically acceptable carrier.

14. A method for treating, preventing, or ameliorating a tumor, comprising administering to a subject in need the antibody or the antigen-binding protein thereof according to claims 1 to 3, the recombinant protein according to claim 4, the antibody-drug conjugate according to claim 9, the immune cell according to claim 11, the multispecific antibody according to claim 12, and the pharmaceutical composition according to claim 13, preferably, the tumor is breast cancer, gastric cancer, pancreatic cancer, ovarian cancer, intestinal cancer, lung cancer, cervical cancer, or other tumor positive for Trop2 expression.

15. The method according to claim 14, which further comprises administration of a second therapeutic agent.

16. Use of the antibody or the antigen-binding protein according to claims 1 to 3, the recombinant protein according to claim 4, the antibody-drug conjugate according to claim 9, the immune cell according to claim 11, the multispecific antibody according to claim 12, and/or the pharmaceutical composition according to claim 13, in preparing a medicament for the prevention and/or treatment of a tumor, wherein the tumor is breast cancer, gastric cancer, pancreatic cancer, ovarian cancer, intestinal cancer, lung cancer, cervical cancer, or other tumor positive for Trop2 expression.

17. A method for detecting the presence or level of Trop-2 in a sample, comprising contacting the sample with the antibody or the antigen-binding fragment thereof according to claims 1 to 3 under conditions allowing formation of a complex by the antibody or the antigen-binding fragment thereof and Trop-2, and detecting the formation of the complex, preferably, the detection is for a non-diagnostic purpose.

18. Use of the antibody or the antigen-binding fragment according to claims 1 to 3 in preparing a detection reagent or kit for Trop-2.

19. A kit comprising the antibody or the antigen-binding protein according to claims 1 to 3, the recombinant protein according to claim 4, the antibody-drug conjugate according to claim 9, the immune cell according to claim 11, the multispecific antibody according to claim 12, and/or the pharmaceutical composition according to claim 13, and optionally instructions for use.

20. An administration device comprising:
(i) an infusion module for administering to a subject a pharmaceutical composition comprising an active ingredient;
(ii) a pharmaceutical composition for infusion, comprising an active ingredient selected from the group consisting of: the antibody or the antigen-binding protein according to claims 1 to 3, the recombinant protein according to claim 4, the antibody-drug conjugate according to claim 9, the immune cell according to claim 11, the multispecific antibody according to claim 12, and/or the pharmaceutical composition according to claim 13; and
(iii) an optional pharmacodynamic monitoring module.
